# EUROPÄISCHE PATENTSCHRIFT

(11) **EP 3 030 569 B1**
(45) Veröffentlichungstag und Bekanntmachung des Hinweises auf die Patenterteilung: **06.05.2020**
(21) Anmeldenummer: 14755041.2
(22) Anmeldetag: 07.08.2014
(51) Int. Cl.: C07F 7/18, C07F 7/08, A61K 6/093, A61K 6/00

(54) **SILANE UND KIESELSÄURE(HETERO)POLYKONDENSATE MIT ÜBER KUPPLUNGSGRUPPEN ANGEBUNDENEN AROMATEN, DIE SICH ALS ODER FÜR MATRIXSYSTEME MIT HOHER TRANSLUZENZ UND GUTER MECHANIK EIGNEN**
SILANE AND SILICIC ACID (HETERO)POLYCONDENSATE HAVING AROMATIC COMPOUNDS LINKED VIA COUPLING GROUPS WHICH ARE SUITABLE AS OR FOR MATRIX SYSTEMS HAVING HIGH TRANSLUCENCY AND GOOD MECHANICAL PROPERTIES
SILANES ET (HÉTÉRO)POLYCONDENSATS D'ACIDE SILICIQUE COMPRENANT DES AROMATES QUI SONT LIÉS PAR DES GROUPES DE LIAISON ET QUI CONVIENNENT COMME OU POUR DES SYSTÈMES MATRICIELS À TRANSLUCIDITÉ ÉLEVÉE ET BONNE MÉCANIQUE

(30) Priorität: 08.08.2013 DE 102013108594
(43) Veröffentlichungstag der Anmeldung: 15.06.2016
(73) Patentinhaber: Wolter, Herbert, 97941 Tauberbischofsheim (DE); Nique, Somchith, 97249 Eisingen (DE)
(72) Erfinder: Wolter, Herbert, 97941 Tauberbischofsheim (DE); Nique, Somchith, 97249 Eisingen (DE)
(74) Vertreter: Strehl Schübel-Hopf & Partner
(86) Internationale Anmeldenummer: PCT/EP2014/067015
(87) Internationale Veröffentlichungsnummer: WO 2015/018906

(56) Entgegenhaltungen:
- WO-A1-2013/041723
- WO-A1-2013/053693
- BURKE S D ET AL: "Synthesis of ethisolide, isoavenaciolide and avenciolide", THE JOURNAL OF ORGANIC CHEMISTRY, AMERICAN CHEMICAL SOCIETY, US, Bd. 57, Nr. 8, 1. Januar 1992 (1992-01-01) , Seiten 2228-2235, XP002971516, ISSN: 0022-3263, DOI: 10.1021/JO00034A009
- DATABASE COMPENDEX [Online] ENGINEERING INFORMATION, INC., NEW YORK, NY, US; 2001, KATO Y ET AL: "Large photoinduced refractive index change of polymer films containing and bearing norbornadiene groups and its application to submicron-scale refractive-index patterning", XP002731562, Database accession no. E2002046840338 & KATO Y ET AL: "Large photoinduced refractive index change of polymer films containing and bearing norbornadiene groups and its application to submicron-scale refractive-index patterning", POLYMER JOURNAL 2001 SOCIETY OF POLYMER SCIENCE JP, Bd. 33, Nr. 11, 2001, Seiten 868-873,
- KATO Y ET AL: "Large Photoinduced Refractive Index Change of Polymer Films Containing and Bearing Norbornadiene Groups and Its Application to Submicron-Scale Refractive-Index Patterning.", POLYMER JOURNAL, vol. 33, no. 11, 1 November 2001 (2001-11-01), pages 868-873, XP055590878, ISSN: 0032-3896, DOI: 10.1295/polymj.33.868

## Beschreibung

Die vorliegende Erfindung betrifft anorganisch-organische Materialien, nämlich Silane, Harze und Matrixsysteme (bei Bedarf mit Füllstoffen versehene, organisch vernetzte oder unvernetzte Kieselsäure(hetero)polykondensate) mit über Kupplungsgruppen daran angebundenen aromatischen Resten, deren (molarer) Anteil genau eingestellt werden kann. Die genannten aromatischen Reste sowie ihr (molarer) Anteil beeinflussen die Brechzahl der ausgehärteten Harze bzw. der aus den Harzen durch organische Polymerisation erzeugten anorganischorganischen Materialien. Durch geeignete Wahl der Substituenten sowie der Menge, mit der sie in die Kieselsäure(hetero)polykondensate eingebaut sind, lässt sich die Brechzahl des Materials genau steuern und damit eine Anpassung der Brechzahl des Materials an die Brechzahl von Füllstoffen erreichen, mit denen es gefüllt werden soll. Mit der Einstellung einer bestimmten Brechzahldifferenz zwischen dem Material und dem Füllstoff lässt sich eine spezifische Transluzenz bzw. gegebenenfalls auch eine spezifische Trübung in gewünschter Weise erreichen, während eine identische oder fast identische Brechzahl des Matrixmaterials und des darin eingebetteten Füllstoffs in der Regel zu einer besonders hohen Transluzenz führt. Auf verschiedenen Gebieten lässt sich ein solches gefülltes Material daher in besonders günstiger Weise einsetzen. Dazu gehört der Dentalbereich, bei dem mit der Einstellung der gewünschten Transluzenz eine speziell geforderte Ästhetik realisiert werden kann, während die Steuerungsmöglichkeit für die Strahlungsdurchlässigkeit einen Einsatz z.B. bei optischen Anwendungen ermöglicht, ohne dass die Erzielung der gewünschten jeweiligen mechanischen Eigenschaften, z.B. eines speziellen E-Moduls, dadurch nachteilig beeinträchtigt wäre. Ein besonderer Vorteil der Systeme liegt darin, dass sich Harze und Matrixsysteme erzeugen lassen, die sich bezüglich der Brechzahl bzw. Transluzenz stufenlos bzw. sehr fein abgestuft einstellen lassen, so dass der Anwender auf eine Vielzahl von Material-/Füllstoff-Kombinationen zugreifen kann, um genau die jeweils gewünschte daraus auszuwählen.

Im Bereich der dentalen Werksstoffe, aber nicht nur dort, ist es wichtig, eine Palette von Materialien bereitstellen zu können, die zwar dem Grunde nach für dieselben Zwecke verwendet werden können und dieselben physikalischen und mechanischen Eigenschaften besitzen, wobei diese Eigenschaften jedoch an spezifische, häufig sogar individuelle Anforderungen detailgenau angepasst werden müssen. Beispiele sind die Farbe bzw. Transluzenz von Überkronungen, die Matrixhydrophilie, die Schrumpfung, die Reaktivität gegenüber Substraten oder weiteren Matrix- oder Komposit-Bestandteilen wie Zahngewebe, Co-Reaktanten oder Reaktionspartnern in lonomer-Kompositen. Hier haben minimale Veränderungen häufig eine große Wirkung. Kann der mit diesen Materialien arbeitende Spezialist, beispielsweise ein Zahnarzt oder ein Zahntechniker, auf eine abgestufte Palette der für seine Zwecke erforderlichen Materialien zurückgreifen, wird er in die Lage versetzt, für jede einzelne Applikation das genau dazu passende Material auszuwählen.

Die Herstellung eines hochästhetischen Zahnersatzes wurde bereits in DE10 2011 054440.2 vorgeschlagen. Die dort offenbarten Materialien sind jedoch teilweise kommerziell nicht verfügbar oder aber relativ teuer in der Herstellung. Es besteht daher ein Bedarf, weitere Materialien bereitzustellen, die ohne die Verwendung der dort eingesetzten Substituenten und Füllstoffe auskommen und sich relativ einfach und kostengünstig in hoher Variabilität herstellen lassen.

In Lösung der genannte Aufgabe werden erfindungsgemäß organisch modifizierte Kieselsäurepolykondensate sowie daraus hergestellte Komposite bereitgestellt, die zu einem gewissen, steuerbaren Anteil eine aromatische Gruppe aufweisen, welche über Stickstoff, Schwefel oder Phosphor an einen über Kohlenstoff an Silicium gebundene Kohlenwasserstoffrest gebunden ist. Die Erfindung stellt auch Silane als Ausgangsmaterialien für diese Kieselsäurepolykondensate bereit. Die erfindungsgemäßen Gegenstände sind in den Ansprüchen 1 bis 15 definiert. In spezifischen Ausführungsformen der Erfindung enthalten die Kieselsäurepolykondensate weiterhin freie Hydroxy- oder Carbonsäuregruppen und/oder C=C-doppelbindungshaltige Reste, die sich einer organischen Polymerisation (Polyaddition) unterwerfen oder über weitere Reaktionen miteinander verbrücken lassen, oder sie enthalten dimere oder oligomere Silanstrukturen. Alle diese Gruppen, Reste und Strukturen ermöglichen weitere Feinabstufungen hinsichtlich der Reaktionsfähigkeit und/oder der Festigkeit der Kondensate und Komposite, wie nachstehend ausführlich erläutert.

Bei den erfindungsgemäßen organisch modifizierten Kieselsäurepolykondensaten kann es sich um ausschließlich aus Silanen aufgebauten Kieselsäurepolykondensaten oder um solche handeln, die neben Silicium andere Metallatome M cokondensierter Verbindungen aufweisen, beispielsweise M = B, Al, Ti, Zn und/oder weitere Übergangsmetallatome, wie dem Grunde nach aus dem Stand der Technik bekannt. Kondensate mit solchen Heteroatomen werden vorliegend als Kieselsäureheteropolykondensate bezeichnet, die Gesamtheit der möglichen Kondensate mit und ohne Heteroatome als Kieselsäure(hetero)polykondensate. Des Weiteren kann es sich um vollständig oder teilweise vernetzte Kondensate von Silanen und ggf. Metallverbindungen, insbesondere Alkoxid-Metallverbindungen, handeln. Wenn die Kondensate nur teilweise vernetzt sind, weisen sie noch an Silicium und/oder Metall gebundene, hydrolytisch kondensierbare Reste oder freie Hydroxygruppen auf, die eine (spätere) weitergehende anorganische Vernetzung unter Ausbildung zusätzlicher Si-O-Si- oder Si-O-M- oder M-O-M-Brücken zulassen. Wird der Ausdruck "Kieselsäurepolykondensat" verwendet, so umfasst dieser Ausdruck anmeldungsgemäß sowohl Hetero- als auch Teil-Kondensate, sofern dies nicht aufgrund des Kontextes ausgeschlossen ist.

Die erfindungsgemäßen Silane besitzen die nachstehende Formel (1). Die erfindungsgemäßen Kieselsäurepolykondensate weisen hydrolytisch kondensierbare Strukturen der nachstehenden Formel (1) auf:

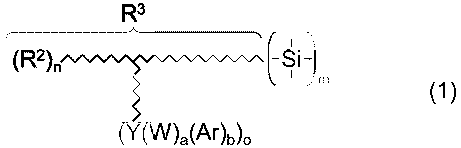

In dieser Formel steht die Zickzacklinie für das Rückgrat eines über ein Kohlenstoffatom an das Silicium gebundenen Kohlenwasserstoffrestes (Substituenten), wobei dieses Rückgrat beliebig durch Heteroatome oder Kupplungsgruppen oder andere, Heteroatome enthaltende Gruppen unterbrochen sein darf. Beispiele sind Unterbrechungen durch -S-, -O-, -NH-, -C(O)O-, -NHCH(O)-, -C(O)NH- -NHC(O)O-, -C(O)NHC(O)-, -NHC(O)NH-, -S(O)-, -C(S)O-, -C(S)NH-, -NHC(S)-, -NHC(S)O- und dergleichen. Da es für die Zwecke der Erfindung auf die Struktur des Rückgrats dieses Restes nicht ankommt, kann der Fachmann diesbezüglich eine beliebige Auswahl treffen. Auch ist die dargestellte Verzweigung des kohlenwasserstoffhaltigen Rückgrats, gezeigt anhand der abzweigenden Zickzacklinie, optional, genauso wie die Möglichkeit einer mehrfachen Verzweigung. Auch die Position der Reste R² und Ar(W)ₐY am Rückgrat ist frei wählbar.

Dieser gesamte, über ein Kohlenstoffatom an das Siliciumatom gebundene Substituent oder Rest ist in Formel (1) mit R³ bezeichnet. Er ist o-fach mit (Ar)_{b}(W)ₐY- substituiert, worin Ar eine Gruppe bedeutet, die mindestens einen aromatischen Rest mit mindestens einer Aryl- und/oder Heteroarylgruppe enthält. Die Aryl- und Heteroarylgruppen können unsubstituiert sein; sie können statt dessen einfach oder mehrfach substituiert sein, wobei die Substituenten vorzugsweise ausgewählt sind unter Alkyl, Alkoxy, Alkylmercapto, Alkoxyalkylen, Alkylthioalkylen, Alkylcarbonyl, Alkylketoalkylen (auch als Alkylcarbonylalkylen zu bezeichnen), Alkylcarboxyl, Alkylcarboxylalkylen, Nitril und Halogen (insbesondere Chlor, Brom, lod). Eine Substitution mit Säuregruppen (z.B. Boronsäure-, Carbonsäure-, Phosphin- oder Phosphonsäure- oder Sulfonsäuregruppen) oder Hydroxygruppen ist dagegen wenig wünschenswert und sollte nach Möglichkeit vermieden werden. Enthält der aromatische Rest mehrere Arylgruppen, so können diese durch andere Gruppen, vorzugsweise ausgewählt unter -R^{x}-, -(R^{x})ₓ-C(O)-(R^{y})_{y}-, -(R^{x})ₓ-S-(R^{y})_{y}-, -(R^{x})ₓ-S(O)-(R^{y})_{y}-, -(R^{x})ₓ-S(O)₂-(R^{y})_{y}-, -(R^{x})ₓ-O-(R^{Y})_{y}- und -(R^{x})ₓ-C(O)₂-(R^{y})_{y}-, unterbrochen sein, worin R^{x} und R^{y} gleich oder verschieden sind und eine vorzugsweise kurzkettige Alkylengruppe (z.B. mit C₁-C₆, darunter -CH₂- und -C(CH₃)₂-) bedeuten und die Indices x und y unabhängig voneinander 0 oder 1 bedeuten Dabei hat in vielen Fällen sowohl x als auch y die Bedeutung 0. Bisphenol-A-, Benzophenon-, Diphenylsulfon-, Diphenylsulfid- oder Diphenylether-Strukturen sind Beispiele hierfür. Alternativ können die vorhandenen Arylgruppen über eine Bindung miteinander verknüpft (wie z.B. im Biphenyl-, Terphenyl- oder Bipyridyl-Rest) oder kondensiert (wie z.B. im Naphthyl-, Anthracen-, Chrysen-Rest) vorliegen.

Die voranstehenden Beispiele betrafen Aromaten ohne Heteroatome im Ring. Als Heteroarylgruppen seien beispielhaft die folgenden genannt: die Thiophen-, Furan-, Pyridin- und die Chinolin-Struktur .

Der Index o hat die Bedeutung 1, 2, 3 oder 4, kann gegebenenfalls aber auch noch größer sein. Bevorzugt ist o = 1 oder 2, ganz besonders bevorzugt 1. Der Index a ist 0, 1 oder 2. Das bedeutet, dass die Gruppe W einfach oder zweifach vorhanden sein oder fehlen kann. W ist eine substituierte oder unsubstituierte Kohlenwasserstoffgruppe, beispielsweise eine Alkylen- oder ein Alkenylengruppe, deren Kohlenstoffkette durch beliebige Heteroatome oder Kupplungsgruppen wie -S-, -O-, -NH-, -NR⁴-, -C(O)O-, -NHC(O)-, -C(O)NH-, -NHC(O)O-, -C(O)NHC(O)-, -NHC(O)NH-, -S(O)-, -C(S)O-, -C(S)NH-, -NHC(S)-, -NHC(S)O- unterbrochen sein kann.

Der Rest Y ist entweder zweibindig und hat dann die Bedeutung -S-, -NR⁴⁻ oder -P(O)(R⁴)_{c}(Z')_{d}-mit Z' = OR⁴, c = 0 oder 1, d = 0 oder 1 und (c+d) = 1 oder er ist dreibindig und bedeutet -N= oder -P(O)=.

Im Falle des Fehlens der Gruppe W sind der oder die Reste Ar direkt an Y angebunden. Dabei können je nachdem, ob Y zweibindig oder dreibindig ist, ein oder zwei Reste Ar an Y angebunden sein. In diesen Fällen muss b daher 1 oder 2 sein. Bei Anwesenheit von W kann b ebenfalls 1 oder 2 sein, kann aber auch größer sein, beispielsweise 3, 4, 5 oder 6 oder sogar eine hoch höhere Zahl bedeuten. Dabei muss mindestens ein Rest Ar an jeder Gruppe W gebunden sein; es können jedoch auch mehrere (zwei oder noch mehr) Reste pro Gruppe W sein.

R⁴ ist in allen vorstehenden Zusammenhängen ein substituierter oder unsubstituierter Kohlenwasserstoffrest, beispielsweise ein Aryl-, Alkylaryl oder Arylalkylrest und vorzugsweise ein substituierter oder - vorzugsweise - unsubstituierter Alkylrest mit stärker bevorzugt 1 bis 6 Kohlenstoffatomen. Außer in der Formel -P(O)(R⁴)_{c}(Z')_{d}- kann R⁴ darüber hinaus in allen diesen Zusammenhängen auch Wasserstoff bedeuten (die Ausnahme gilt auch für die Definition Z' = OR⁴; auch hier kann R⁴ nicht Wasserstoff bedeuten).

Der Substituent oder Rest R³ kann weiterhin mit dem Rest R² substituiert sein. Bei diesem Rest handelt es sich um einen reaktiven Substituenten, ausgewählt unter einer Hydroxygruppe, einem freien Carbonsäurerest -COOH oder einem Ester oder einem Salz davon. Dieser Rest muss nicht notwendigerweise vorhanden sein (n = 0), oder er kann einfach oder mehrfach (n = 1, 2, 3, 4, oder >4) vorliegen. Im Falle seines mehrfachen Vorhandenseins können die Reste R² identisch oder auch verschieden sein. In Kieselsäurepolykondensaten können auch unterschiedliche Reste R³ vorliegen, von denen nur ein Teil einen, ggf. auch mehrere Reste R² trägt. Mehrere Reste R² an einem Rest R³ können sich an beliebigen Positionen des Kohlenwasserstoffgerüsts von R³ befinden; Bedingung ist nur, dass sie an ein Kohlenstoffatom gebunden sind.

Die vorstehend erläuterten Variationsmöglichkeiten von R² an den Substituenten oder Resten R³ trägt insbesondere ganz wesentlich zu einer wunschgemäß einstellbaren Abstufbarkeit des Vernetzungsgrades bei, wobei diese Abstufbarkeit unabhängig von der ebenfalls abstufbaren Einstellung der Brechzahl ist, wie nachstehend näher erläutert wird.

Der Substituent oder Rest R³ kann, muss aber nicht, neben dem gezeigten Rest R² zusätzliche, ggf. dem Grunde nach ebenfalls reaktive Substituenten tragen, die dann aber für die vorliegende Erfindung keine Rollen spielen.

Die drei nicht näher gekennzeichneten Bindungen des Si-Atoms stehen dann, wenn es sich bei (1) um ein Silan handelt, für weitere am Siliciumatom gebundene Reste wie aus dem Stand der Technik bekannt. Stattdessen können sie zumindest teilweise oder aber in vollem Umfang Sauerstoffbrücken zu weiteren Siliciumatomen bzw. anderen Metallatomen symbolisieren, wenn die Struktur (1) Bestandteil eines Kieselsäure(hetero)polykondensats ist. Da die zur Erfindung führenden Reaktionen sowohl an monomeren Silanen als auch an bereits anorganisch vernetzten Kieselsäurepolykondensaten erfolgen können, kommt es auf die Natur dieser Bindung nicht an, denn im Falle von Silanen können diese nachträglich einer hydrolytischen Kondensation zugeführt und damit in ein Kieselsäurepolykondensat überführt werden.

Als erste Gruppe der weiteren am Siliciumatom gebundenen Reste von Silanen sind über Kohlenstoff am Siliciumatom gebundene Reste zu nennen. Hierfür eignen sich beliebige Reste, darunter Alkyl- (vorzugsweise C₁-C₆-Alkyl) und Alkenylreste. Diese können substituiert oder unsubstituiert sein. In spezifischen Fällen handelt es sich bei ihnen um einen (und ganz selten sogar zwei) weitere(n) Substituenten oder Rest(e) R³ wie oben definiert. Als zweite Gruppe sind unter Hydrolysebedingungen hydrolysierbare Gruppen zu erwähnen. Diese sind dem Fachmann aus einer Vielzahl von Publikationen bekannt, weshalb sie hier nicht näher erläutert werden müssen. Sie umfassen neben über ein Sauerstoffatom an das Silicium gebundenen Kohlenwasserstoffresten, darunter Alkoxiden (vorzugsweise C₁-C₆-Alkoxy) und Acylresten Halogenatome wie Cl oder Br, Aminogruppen und dergleichen. Neben Substituenten oder Resten aus diesen beiden Gruppen können sowohl die erfindungsgemäßen Silane als auch die erfindungsgemäßen Kieselsäurepolykondensate gegebenenfalls auch freie OH-Gruppen tragen.

In den Silanen ist das Verhältnis von über Kohlenstoff am Siliciumatom gebundenen Resten zu hydrolytisch kondensierbaren Resten oder OH im Grunde nicht kritisch; der Fachmann weiß, dass im Falle von drei hydrolytisch kondensierbaren Resten bzw. OH-Gruppen ein sehr dichtes anorganisches Netzwerk entsteht, während im Falle von zwei solchen Resten bzw. OH-Gruppen vorwiegend Ketten und Ringe gebildet werden. Beim Vorhandensein von nur einem solchen Rest / einer OH-Gruppe können nur Dimere gebildet werden. Es werden daher je nach Anwendungszweck geeignete Varianten bzw. Kombinationen hierunter ausgewählt; in der Regel sind Ausgangssilane mit 2 hydrolytisch kondensierbaren Resten bzw. OH-Gruppen gut für die Erfindung geeignet.

Der Index m ist 1 oder 2, gegebenenfalls aber auch 3, 4 oder noch größer. Eine Beschränkung nach oben ist theoretisch nicht gegeben. Wenn m > 1 ist, enthält der kohlenwasserstoffhaltige Rest R³ mehr als eine Silylgruppe.

Wie oben erwähnt, hat der Index o die Bedeutung 1, 2, 3 oder 4, kann gegebenenfalls aber auch noch größer sein. Für den Fall, dass o 2 oder größer 2 ist, können die zwei oder mehr Reste Y(W)ₐ(Ar)_{b} im Rahmen der vorstehend angegebenen Definition eine unterschiedliche Bedeutung besitzen. Es ist jedoch bevorzugt, dass o = 1 ist. Mehrere Reste Y(W)ₐ(Ar)_{b} können an beliebigen Kohlenstoffatomen der Struktur (1) bzw. des Silans (1) angebunden sein.

Im Falle von Kieselsäurepolykondensaten ist es auch möglich, dass in einem Teil der Substituenten oder Reste R³ o = 1 oder größer 1 wie oben angegeben ist, während in einem anderen Teil der Substituenten oder Reste R³ o = 0 ist, der Rest Y(W)ₐ(Ar)_{b} also fehlt. Es ist bevorzugt, dass in solchen Fällen in mindestens etwa jedem fünften Rest R³ o = 1 oder größer ist, dass o über die Zahl der Substituenten oder Reste R³ hinweg gesehen durchschnittlich also mindestens 0,2 beträgt. Stärker bevorzugt ist, dass wenigstens jeder zweite Rest R³ einen (oder mehrere) Reste Y(W)ₐ(Ar)_{b} trägt, dass o also einen Wert zwischen 0,5 und 1 besitzt. Er kann aber auch größer 1 sein, z.B. 1,5 oder 2,0 oder sogar darüber. Auch in allen diesen Ausführungsformen können unterschiedliche Substituenten oder Reste R³ mit unterschiedlichen Resten Y(W)ₐ(Ar)_{b} substituiert sein wie oben für den Fall angegeben, dass ein Substituent oder Rest R³ mehr als einen solchen Rest aufweist, dass also o = oder > 2 ist. Die Erfindung umfasst ferner Gemische von Silanen mit unterschiedlichen Substituenten oder Resten R³, wobei in den unterschiedlichen Resten R³ der Index o unterschiedlich ist, die Reste R³ also keine, eine oder sogar mehrere Reste Y(W)ₐ(Ar)_{b} aufweisen können.

Wenn nicht jeder Substituent R³ im Kieselsäurepolykondensat einen (oder mehrere) Reste (Ar)_{b}(W)ₐY- aufweist, weisen diejenigen Substituenten R³, die frei von diesem Rest sind oder nicht die maximale Anzahl davon besitzen, in der Regel statt dessen denjenigen Rest auf, durch dessen Substitution der Rest (Ar)_{b}(W)ₐY- gebildet wurde. Dieser wird nachstehend als R¹ bezeichnet. Geeignete Reste R¹ werden bei der nachstehenden Diskussion der Auswahl der Ausgangsmaterialien ausführlich erläutert; sie enthalten einen nichtaromatischen C=C-doppelbindungshaltigen Rest.

Das Vorhandensein des oder der aromatischen Reste des Substituenten (Ar)_{b}(W)ₐY- bewirkt eine Verschiebung, insbesondere eine Erhöhung des Brechungsindex sowohl des Kieselsäurepolykondensats als auch des ausgehärteten Kieselsäurepolykondensats, relativ zu entsprechenden Verbindungen, in denen dieser Substituent fehlt.

Da die Erfindung Kondensate bereitstellt, deren Gehalt an aromatischen Resten unter anderem über die Einstellung des Substitutionsgrades (Wert des Index o) sowie über die Wahl der Anzahl der Reste R³ im Kondensat reguliert werden kann, stellt die Erfindung ein Material bereit, dessen Brechungsindex auf einfache Weise je nach Bedarf eingestellt werden kann. Aufgrund der Tatsache, dass die Anzahl der Reste (Ar)_{b}(W)ₐY- unabhängig von der Anzahl der Reste R² ist und diese Reste ihrerseits (a) in frei wählbaren Anteilen vorhanden sein und (b) in frei wählbaren Relationen auch wieder in Reste R¹ überführt werden können, die organisch polymerisierbare C=C-Doppelbindungen tragen, wie weiter unten ausführlich erläutert, stellt die Erfindung Materialien bereit, in denen sich der Brechungsindex und die mechanischen Eigenschaften unabhängig voneinander beliebig und sehr fein einstellen lassen.

Die erfindungsgemäßen Silane der Formel (1) sowie die Kieselsäure(hetero)polykondensate, die Strukturen der Formel (1) enthalten, sind ausgehend von bekannten Silanen erhältlich. Diesen Silanen ist wie bereits oben angesprochen gemeinsam, dass sie einen über Kohlenstoff an Silicium gebundenen Rest R¹ aufweisen, der mindestens eine nichtaromatische C=C-Doppelbindung enthält. Diese Doppelbindungen können beispielsweise als ein Bestandteil von Vinyl- oder Allylgruppen oder von einer Michael-Addition zugänglichen Gruppen wie (Meth)Acryl- oder Norbornenylgruppen vorliegen. Beispiele für derartige Silane lassen sich den folgenden Druckschriften entnehmen: DE 40 11 044 A1, EP 450 624, DE 44 16 857 C1, DE 196 27198, DE199 10 895 A1, DE 103 49 766 A1, DE 101 32 654 A1, DE 10 2005 016 059 A1, DE 102011 054 440 A1, DE 10 2011 053 865 A1 und DE 10 2012 109 685 A1 aufgeführt. Neben Silanen mit einem geradkettigen oder verzweigten organischen Rest, der eine oder zwei C=C-Doppelbindungen und 5 bis 50 Kohlenstoffatomen trägt und über eine Gruppierung A mit A = O, S, PR", POR" oder NHC(O)O an den über Kohlenstoff an das Silicium gebundenen Rest verknüpft ist wie in DE 40 11 044 A1 offenbart oder worin die genannte Verknüpfung über eine Säureamid-Gruppe erfolgt wie in DE 199 10 895 A1 offenbart, können das beispielsweise Silane sein, die ähnlich aufgebaut sind wie die vorgenannten, jedoch zusätzlich eine Hydroxy- oder COOH-Gruppe wie in DE 44 16 857 C1 offenbart oder eine Phosphor enthaltende Gruppe, beispielsweise eine Phosphonsäure aufweisen wie aus DE 101 32 654 A1 bekannt. Andere Beispiele sind in DE 103 49 766 genannt; diese Silane weisen mindestens zwei gleiche oder unterschiedliche C=C-doppelbindungshaltige Reste auf, die in unterschiedlichen Abständen zum Siliciumatom am über Kohlenstoff gebundenen Kohlenwasserstoffrest angebunden sind, wobei mindestens eine der C=C-doppelbindunghaltigen Reste über eine Kopplungsgruppe -NH-C(O)O-, -NH-C(O)- oder -CO(O)- mit dem Kohlenwasserstoffrest verknüpft ist. Und schließlich sei auf Silane verwiesen, die mindestens eine Norbornenyl- oder verwandte Gruppierung enthalten, wie in DE 196 27 198 A1 erläutert.

Der Rest R¹ enthält in der Regel mindestens zwei und vorzugsweise bis zu ca. 50, ggf. aber auch noch mehr Kohlenstoffatome. Die organisch polymerisierbare Gruppe kann dabei direkt oder über eine Kupplungsgruppe an das Kohlenstoffskelett des kohlenwasserstoffhaltigen Restes gebunden sein. Bevorzugt besitzt der organisch polymerisierbare Rest mindestens eine C=C-Doppelbindung, häufig auch zwei C=C-Doppelbindungen. In spezifischen Ausführungsformen ist er oder weist er mindestens eine Acrylat- oder Methacrylatgruppe auf.

Als beispielhafte Silane bzw. Kieselsäure(hetero)polykondensate seien die nachstehend aus dem Stand der Technik bekannten genannt, wobei die Definitionen der Reste und Indices den jeweiligen Druckschriften entnommen und nicht mit den für die Beschreibung der vorliegenden Erfindung verwendeten gleichzusetzen sind:
Silane der allgemeinen Formel (A):

   {XₐR_{b}Si[R'(A)_{c}]_{(4-a-b)}}ₓB (A)

   worin die Reste folgende Bedeutung haben:
   X: Hydroxy, Alkoxy, Acyloxy, Alkylcarbonyl, Alkoxycarbonyl oder-NR"₂;
   R: Alkyl, Alkenyl, Aryl, Alkylaryl oder Arylalkyl;
   R': Alkylen, Arylen oder Alkylenarylen;
   R": Wasserstoff, Alkyl oder Aryl;
   A: O, S, PR", POR" oder NHC(O)O;
   B: geradkettiger oder verzweigter organischer Rest, der sich von einer Verbindung mit mindestens drei C=C-Doppelbindungen und 5 bis 50 Kohlenstoffatomen ableitet;
   a: 1, 2 oder 3;
   b: 0, 1 oder 2;
   c: 0 oder 1;
   x: ganze Zahl, deren Maximalwert der Anzahl von Doppelbindungen in der Verbindung B minus 1 entspricht,
   sowie davon abgeleitete Kieselsäurepolykondensate, entstanden durch hydrolytische Kondensation der Silane der Formel (A). Solche Silane und Polykondensate sind in DE 40 11 044 A1 offenbart. Sie fallen unter die vorliegende Strukturformel (1) mit R¹ und R² gleich organisch polymerisierbarem Rest.
Silane der allgemeinen Formel (B):

   B{A-(Z)_{d}R¹(R²)-R'-SiXₐR_{b}}_{c} (B)

   worin die Reste und Indices folgende Bedeutung haben:
   A = O, S, NH oder C(O)O;
   B = ein geradkettiger oder verzweigter organischer Rest mit mindestens einer C=C-Doppelbindung und 4 bis 50 Kohlenstoffatomen;
   R = Alkyl, Alkenyl, Aryl, Alkylaryl oder Arylalkyl;
   R' = Alkylen, Arylen, Arylenalkylen oder Alkylenarylen mit jeweils 0 bis 10 Kohlenstoff-Atomen, wobei diese Reste durch Sauerstoff- und Schwefelatome oder durch Aminogruppen unterbrochen sein können;
   R¹ = Stickstoff, Alkylen, Arylen oder Alkylenarylen mit jeweils 1 bis 10 Kohlenstoffatomen, wobei diese Reste durch Sauerstoff- oder Schwefelatome oder durch Aminogruppen unterbrochen sein können;
   R² = OH oder COOH;
   X = Hydroxy, Alkoxy, Acyloxy, Alkylcarbonyl, Alkoxycarbonyl oder-NR"₂;
   R" = Alkyl oder Aryl;
   Z = CO oder CHR, mit R gleich H, Alkyl, Aryl oder Alkylaryl;
   a = 1, 2 oder 3;
   b = 0,1 oder 2;
   sowie davon abgeleitete Kieselsäurepolykondensate, entstanden durch hydrolytische Kondensation der Silane mit der Formel (B). Solche Silane und Kieselsäurepolykondensate sind in DE 44 16 857 C1 offenbart. Sie repräsentieren Strukturen der Formel (1), worin R² eine Hydroxygruppe oder eine Carbonsäuregruppe ist.
Silane der allgemeinen Formel (C) 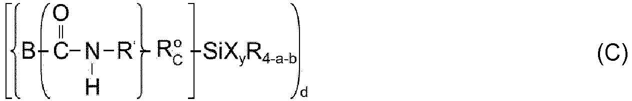 worin die Reste und Indices die folgende Bedeutung haben:
   B = organischer Rest mit mindestens einer C=C-Doppelbindung;
   R = Alkyl, Alkenyl, Aryl, Alkylaryl oder Arylalkyl;
   R° und R' jeweils = Alkylen, Alkenylen, Arylen, Alkylenarylen oder Arylenalkylen;
   X = Hydroxy, Alkoxy, Acyloxy, Alkylcarbonyl, Alkoxycarbonyl oder -NR"₂ mit R" gleich Wasserstoff, Alkyl oder Aryl;
   a = 1, 2 oder 3
   b = 1, 2 oder 3, mit a + b = 2, 3 oder 4;
   c = 1;
   d = 1, 2, 3, 4, 5, 6, 7, 8, 9 oder 10;
   e = 2 oder 3 oder 4;
   sowie davon abgeleitete Kieselsäurepolykondensate, entstanden durch hydrolytische Kondensation von Silanen mit der Formel (C). Die Silane der Formel (C) sowie die daraus ableitbaren Kieselsäurepolykondensate sind in DE 199 10 895 A1 offenbart. Sie fallen unter Strukturen der Formel (1), worin R² ein organisch polymerisierbarer Rest ist. Für den Fall, dass b in der Formel (C) 2 oder 3 bedeutet, haben auch einer oder zwei der weiteren Bindungen des Siliciumatoms die Bedeutung von R³ In Formel (1).
Silane der allgemeinen Formel (D):

   {B'-Z'-R¹(B)-R-}ₐ(R')_{b}SiX_{4-a-b} (D)

   worin die Reste und Indices die folgende Bedeutung haben:
   R ist eine Alkylen-, Arylen- oder Alkylenarylengruppe, die durch eine oder mehrere Sauerstoff- oder Schwefelatome oder Carboxyl- oder Aminogruppen unterbrochen sein oder solche Atome/Gruppen an ihrem dem Siliciumatom abgewandten Ende tragen kann;
   R¹ ist eine mit Z' substituierte Alkylen-, Arylen- oder Alkylenarylengruppe, die durch eine oder mehrere Sauerstoff- oder Schwefelatome oder Carboxyl- oder Aminogruppen unterbrochen sein oder solche Atome/Gruppen an einem ihrer Enden tragen kann;
   R' ist eine Alkyl-, Alkenyl-, Aryl-, Alkylaryl- oder Arylalkylgruppe;
   B und B' können gleich oder verschieden sein, beide Reste haben die Bedeutung einer geradkettigen oder verzweigten organischen Gruppe mit mindestens einer C=C-Doppelbindung und mindestens zwei Kohlenstoffatomen;
   X ist eine Gruppe, die unter Ausbildung von Si-O-Si-Brücken eine hydrolytische Kondensationsreaktion eingehen kann, mit Ausnahme von Wasserstoff und Halogen;
   Z' hat die Bedeutung -NH-C(O)O-, -NH-C(O)- oder -CO(O)-, wobei die beiden erstgenannten Reste über die NH-Gruppe am Rest B' gebunden sind, während die Carboxylatgruppe in beiden Richtungen weisen kann;
   a bedeutet 1 oder 2 und
   b ist 0 oder 1;
   sowie daraus abgeleitete Kieselsäurepolykondensate, die durch hydrolytische Kondensation der Silane (D) erhalten werden können. Solche Silane und Polykondensate sind in DE 103 49 766 A1 offenbart; sie lassen sich unter die Struktur (1) subsumieren, worin R² ein organisch polymerisierbarer Rest ist.
Silane der allgemeinen Formel (E):

   (XₐR_{b}Si)ₘ[-{B}-([O]ₒP[O]ₚR'_{c}Y_{d})ₙ]_{4ab} (E)

   worin die Gruppen, Reste und Indices die folgende Bedeutung haben:
   B ist eine mindestens zweibindige, geradkettige oder verzweigte Gruppe mit mindestens einem organisch polymerisierbaren Rest und mindestens 3 Kohlenstoffatomen,
   X ist ein vom Siliciumatom abhydrolysierbarer Rest oder OH, mit Ausnahme von Wasserstoff und Halogen,
   R und R' sind unabhängig voneinander ggf. substituiertes Alkyl, Alkenyl, Aryl, Alkylaryl oder Arylalkyl,
   Y ist OH oder OR',
   a ist 0, 1, 2 oder 3,
   b ist 0, 1 oder 2, wobei a + b zusammen 1, 2 oder 3 sind,
   c ist 0, 1 oder 2,
   d ist 0, 1 oder 2,
   c + d sind zusammen 2,
   m ist mindestens 1, mit der Maßgabe, dass m nicht mehr als 1 ist, wenn a + b 1 oder 2 bedeutet,
   n ist mindestens 1,
   o ist 0 oder 1, und
   p ist 0 oder 1,
   sowie daraus abgeleitete Kieselsäurepolykondensate, die durch hydrolytische Kondensation der Silane mit der Formel (E) erhalten werden. Silane der Formel (E) und daraus abgeleitete Kieselsäurepolykondensate sind in DE 101 32 654 A1 offenbart. Sie fallen unter die Struktur (A), worin R² ein phosphorhaltiger Rest, beispielsweise eine Phosphonsäure ist.
Silane der allgemeinen Formel (F): 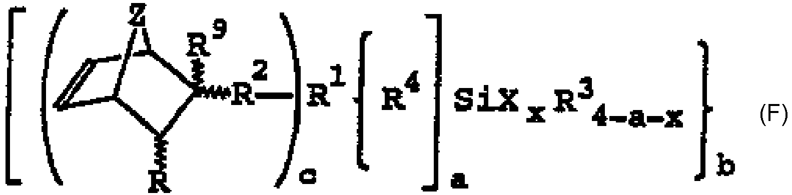 worin die Reste und Indices gleich oder verschieden sind und folgende Bedeutung haben:
   R ist Wasserstoff, R²-R¹-R⁴-SiXₓR³₃₋ₓ, Carboxyl, Alkyl, Alkenyl, Aryl, Alkylaryl oder Arylalkyl,
   R¹ und R² sind unabhängig voneinander Alkylen, Arylen, Arylenalkylen oder Arylenalkylen,
   R³ ist Alkyl, Alkenyl, Aryl, Alkylaryl oder Arylalkyl,
   R⁴ ist -(CHR⁶-CHR⁶)ₙ- mit n = 0 oder 1, CHR⁶-CHR⁶-S-R⁵-, -C(O)-S-R⁵-, CHR⁶-CHR⁶-NR⁶-R⁵, -Y-C(S)-NH-R⁵, -S-R⁵, -Y-C(O)-NH-R⁵-, -C(O)-O-R⁵-, -Y-CO-C₂H₃(COOH)-R⁵-, -Y-CO-C₂H₃(OH)-R⁵- oder -C(O)-NR⁶-R⁵,
   R⁵ ist Alkylen, Arylen, Arylenalkylen oder Arylenalkylen,
   R⁶ ist Wasserstoff, Alkyl oder Aryl mit 1 bis 10 Kohlenstoffatomen,
   R⁹ ist Wasserstoff, Alkyl, Alkenyl, Aryl, Alkylaryl oder Arylalkyl,
   X ist Hydroxy, Alkoxy, Acyloxy, Alkylcarbonyl oder Alkoxycarbonyl;
   Y ist -O-, -S- oder NR⁶,
   Z ist -O- oder -(CHR⁶)ₘ mit m gleich 1 oder 2;
   a ist 1, 2 oder 3, mit b = 1 für a = 2 oder 3
   bist 1, 2 oder 3, mit a = 1 für b = 2 oder 3
   c ist eine ganze Zahl von 1 bis 6,
   x ist 1, 2 oder 3 und
   a + x sind 2, 3 oder 4.
mit der Bedingung, dass dann, wenn c = 1 ist, R⁴ -Y-CO-C₂H₃(COOH)-R⁵- oder -Y-CO-C₂H₃(OH)-R⁵- sein muss. Derartige Silane sowie daraus hergestellte Kieselsäurepolykondensate sind aus DE 196 27198 bekannt.

Die erfindungsgemäßen Silane oder Strukturen der Formel (1) lassen sich erzeugen, indem ein Silan der Formel (0) oder ein Kieselsäure(hetero)polykondensat mit der Struktur (0) mit einem über Kohlenstoff an Silicium gebundenen Rest R¹, der mindestens eine nichtaromatische C=C-Doppelbindung enthält, umgesetzt wird mit einer Verbindung (II), wobei die Umsetzung der folgenden Reaktionsgleichung gehorcht:

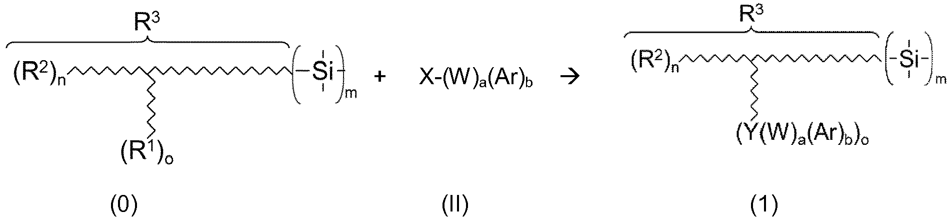

Der Rest R¹ enthält in der Regel mindestens zwei und vorzugsweise bis zu ca. 50, ggf. aber auch noch mehr Kohlenstoffatome und besitzt mindestens eine C=C-Doppelbindung, und stärker bevorzugt ist er einer Michael-Addition zugänglich. In spezifischen Ausführungsformen ist er oder weist er mindestens eine (Meth)Acryl-, insbesondere eine Acrylat- oder Methacrylatgruppe auf. Der Rest R¹ kann direkt oder über eine Kupplungsgruppe an das Kohlenstoffskelett des kohlenwasserstoffhaltigen Restes R³ gebunden sein.

Der Ausdruck "(Meth)Acryl..." soll vorliegend bedeuten, dass es sich jeweils um die entsprechende Acryl- oder die entsprechende Methacryl-Verbindung oder ein Gemisch von beiden handeln kann. Die vorliegenden (Meth)Acrylsäure-Derivate umfassen die Säuren selbst, ggf. in aktivierter Form, Ester, Amide, Thioester und dgl.

In Formel (II) besitzen W, Ar, a und b die oben angegebenen Bedeutungen, und X ist ausgewählt ist unter HS-, HNR⁴- und HP(O)ₑR⁴_{c}Z'_{d}-, wobei Z', R⁴, e, c und d die oben für Formel (1) angegebenen Bedeutungen besitzen, HN= und HP(O)=. Als Beispiele für einsetzbare Verbindungen (II) seien die folgenden genannt:
Thiophenol, 1-Naphthalenthiol, 2-Naphthalenthiol, 4-Phenylthiophenol, 4-Terphenylthiol, 2-, 3- und 4-Thiokresol, N-Methyl-4-biphenylamin, N-Methyl-1-naphthylamin, N-Methyl-2-naphthylamin, Carbazol, 4-(3-Thienyl)-anilin, 2-Aminoanthracen, 2-Thiophenmethylamin, Anilin, 4- Phenylsulfanylanilin, 4-Benzylanilin, 1-Aminonaphthalin, 2-Aminonaphthalin, 6-Aminochrysen, 2-, 3- und 4-Aminobenzophenon, 3,- 5- und 6-Aminochinolin, Pyrrol, 3- bzw. 4-Aminoacetophenon, Phosphonsäurederivate wie Diphenylphosphit H-P(O)(OPh)₂ oder Ethylphenylphosphinat H-P(O)(OEt)(Ph) und Phosphinoxide wie Diphenylphosphinoxid H-P(O)(Ph)₂. Bei der Umsetzung greift die SH-, NH- bzw. PH-Gruppe an der Doppelbindung an (ersteres ist die bekannte Thiol-en-Addition), wodurch der Rest -(W)ₐ(Ar)_{b} über die entstehende Gruppierung Y an die dabei entstehende Struktur (I) angebunden wird.

Diese Reaktion kann stöchiometrisch erfolgen, also mit einem Mol der Verbindung (II) pro Mol Rest R¹. Sie kann aber auch unterstöchiometrisch erfolgen, also mit weniger als einem Mol der Verbindung (II) pro Mol Rest R¹. Im Falle von Silanen erhält man dabei ein Silangemisch aus dem Ausgangssilan der Formel (0), das weiterhin den Rest R¹ trägt, in Kombination mit dem Produkt-Silan (1). Aus diesem Silan oder Silangemisch lässt sich durch hydrolytische Kondensation ein Kieselsäurepolykondensat erzeugen, das im Falle des Gemischs sowohl Strukturen der Formel (0) als auch solche der Formel (1) aufweist. Durch den Anteil an Verbindung (II) bei der Umsetzung kann daher sehr fein eingeregelt werden, wie hoch der Anteil an Ar im gebildeten Kieselsäure(hetero)polysiloxan ist, so dass sich die oben hierfür angegebenen Werte in vollem Umfang realisieren lassen. Diese Einregelung kann alternativ auch dadurch erfolgen, dass Silane der Formel (1) mit Silanen cokondensiert werden, die keine Gruppen Ar im obigen Sinne enthalten, wobei sie z.B. die Formel (0) aufweisen, gegebenenfalls aber auch eine etwas oder völlig andere Struktur besitzen können, z.B. Silane der Formel (4)

R'ₐR"_{b}SiX_{4-a+b} (4)

worin
R' eine unsubstituierte oder substituierte Alkyl- oder Alkenylgruppe mit vorzugsweise 1 bis 12 Kohlenstoffatomen bedeutet,
R" ein substituierter oder unsubstituierter Kohlenwasserstoffrest ist, der eine organisch polymerisierbare oder eine nicht organisch polymerisierbare C=C-Doppelbindung aufweisen kann und beispielsweise eine Alkylen- oder eine Alkenylengruppe mit vorzugsweise 2 bis 20 Kohlenstoffatomen ist oder enthält und/oder dessen Kohlenstoffkette durch beliebige Heteroatome oder Kupplungsgruppen wie -S-, -O-, -NH-, -NR⁴-, -C(O)O-, -NHC(O)-, -C(O)NH-, -NHC(O)O-, -C(O)NHC(O)-, -NHC(O)NH-, -S(O)-, -C(S)O-, -C(S)NH-, -NHC(S)- oder -NHC(S)O- einfach oder mehrfach unterbrochen ist, und
X eine unter Hydrolysebedingungen hydrolysierbare Gruppe wie aus dem Stand der Technik bekannt ist, die ausgewählt sein kann unter über ein Sauerstoffatom an das Silicium gebundenen Kohlenwasserstoffresten, darunter Alkoxiden (vorzugsweise C₁-C₆-Alkoxy) und Acylresten, Halogenatome wie Cl oder Br, Aminogruppen und dergleichen, oder worin X eine freie OH-Gruppe sein kann,
a = 0, 1, 2 oder 3 und
b = 0, 1, 2 oder 3 mit der Maßgabe, dass a+b zusammen = 1, 2 oder 3 ist

Je nach Verhältnis des Silans der Formel (1) zu dem oder den weiteren Silan(en) der Formel (0) oder (4) oder dergleichen kann damit ein Kieselsäure(hetero)polysiloxan erzeugt werden, das zwischen >1 und ≤100 Mol-% (für b = 1), ggf. auch bis zu ≤200 Mol-% (für b = 2) oder noch mehr (für b = 3 oder größer) Gruppen Ar pro Silicium-Atom aufweist.

Wenn die Umsetzung bereits auf der Ebene der Kieselsäure(hetero)polykondensate abläuft, können diese entweder ausschließlich aus Struktureinheiten (1) aufgebaut sein oder aber einen beliebigen Anteil an Struktureinheiten (0) besitzen, der z.B. aus einem unterstöchiometrischen Einsatz der Verbindung (II) resultiert. Dieser beliebige Anteil kann bis zu knapp einem Substituenten oder Rest R³ pro Siliciumatom betragen oder sogar noch größer sein; letzteres aber nur dann, wenn manche oder alle Siliciumatome zwei Reste R³ besitzen. Er kann jedoch auch darunter liegen, derart, dass nicht alle oder nur ein Anteil von z.B. 25, 50 oder 75 Mol-% der Siliciumatome einen Rest R³ der Struktur (0) aufweist. Durch die Wahl der Menge an Verbindung (II) (stöchiometrisch oder unterstöchiometrisch, bezogen auf die Menge an Resten R², z.B. 2, 10, 50 oder 100 Mol-%)) sowie der Anzahl an Gruppen Ar pro Verbindung (II) kann eingeregelt werden, wie hoch der Anteil an Gruppen Ar sein soll.

Ein besonderer Vorzug der vorliegenden Erfindung liegt also darin, dass sich bei geeigneter Wahl eines Ausgangsmaterials, das ja aus dem Stand der Technik zur Verfügung steht, mit nur einer Umsetzung beliebige Abstufungen erzielen lassen, denn mit der Wahl des Ausgangsmaterials (Kieselsäurepolysiloxan der Formel (0) oder entsprechendes Silan/ Silangemisch) lässt sich bereits der Anteil an Gruppen R¹ pro Siliciumatom vorgeben. Mit der Wahl des stöchiometrischen Verhältnisses von Verbindung (II) zu Rest R³ sowie der Menge an Gruppen Ar pro Verbindung (II) lässt sich dann die Relation von Siliciumatomen zur Anzahl der Gruppen Ar, die Relation von Siliciumatomen zur Anzahl der verbliebenen Reste R¹ sowie die Relation der Anzahl der Gruppen Ar zur Anzahl der verbliebenen Reste R¹ völlig frei festlegen. Dabei hat die Umsetzung auf der Ebene der Kieselsäurepolykondensate den Vorteil, dass man ausgehend von einem einzigen Kondensat und mit nur jeweils einem Schritt pro hergestelltem Kondensat zu einer fein abgestuften Produktpalette gelangen kann.

Bei den nichtaromatischen C=C-Doppelbindungen des Restes R¹ handelt es sich um Gruppierungen, die unter dem Einfluss von Wärme, Licht, ionisierender Strahlung oder redoxinduziert (z.B. mit einem Initiator (Peroxid oder dgl.) und einem Aktivator (Amin oder dgl.)) einer organischen Vernetzung unterworfen werden und dabei in Polymere übergehen (engl.: addition polymerization oder chain-growth polymerization) können. Unter dem Attribut "polymerisierbar" bzw. dem entsprechenden Substantiv "Polymerisation" ist daher vorliegend eine entsprechende Polyreaktion zu verstehen, bei der weder Abspaltungen von molekularen Bestandteilen auftreten, noch Wanderungen oder Umlagerungen. Beispiele für R¹ sind einer Michaeladdition zugängliche Doppelbindungen wie Styryle oder (Meth)Acrylsäure-Derivate; es kann sich aber auch um Vinyl- oder Allylgruppen handeln.

In allen Fällen, in denen die Umsetzung mit einer geringeren molaren Menge an (II) erfolgt als Reste R¹ vorhanden sind und die Kieselsäure(hetero)polykondensate bzw. (für den Fall, dass mit Silanen gearbeitet wird, die noch nicht hydrolytisch kondensiert wurden) Silanmischungen daher noch unumgesetzte nichtaromatisch C=C-Doppelbindungen enthalten, können diese ggf. später für andere Reaktionen, insbesondere aber für eine organische Vernetzung des Kieselsäurepolykondensats genutzt werden, die im Falle des Einsatzes von Silanen vorzugsweise erst nach deren Polykondensation erfolgt. Eine solche Polymerisationsreaktion macht eine z.B. lichtinduzierte nachträgliche Vernetzung des Kieselsäurepolykondensats möglich, mit der beispielsweise im Rahmen einer Dentalapplikation (z.B. bei Einsatz eines Zahn-Restaurationsmaterials) das eingesetzte Kieselsäure(hetero)polykondensat bzw. ein Komposit aus diesem Kondensat als Matrix und darin eingebetteten Füllpartikeln nach Abschluss der Formgebung im Mund des Patienten gehärtet werden kann. In einer Ausführungsform der Erfindung ist es aus diesem Grunde bevorzugt, eine unterstöchiometrische Menge an Verbindung (II) einzusetzen und ggf. die gewünschte Menge an aromatischen Gruppen Ar über einen entsprechend hohen Anteil daran in Verbindung (II) einzuführen. Es gibt jedoch auch noch andere Wege, um eine nachträgliche organische Vernetzung des erfindungsgemäßen Kieselsäurepolykondensats zu ermöglichen, was weiter unten dargestellt wird.

Denn es kann häufig günstig sein, den Anteil an Verbindung (II) nicht zu niedrig zu wählen. Der Grund hierfür liegt darin, dass bei der Addition von hohen Anteilen an (II) neben dem Brechzahlanstieg nicht nur der erwartete E-Modulabfall, sondern überraschenderweise auch eine flexibilisierende Wirkung auf die gehärteten Materialien beobachtet wird.

Außerdem konnten die Erfinder den überraschenden Effekt beobachten, dass in solchen Fällen, in denen mit einer Thiolverbindung als Verbindung der Formel (II) gearbeitet wird, eine matrixinterne Stabilisierung durch die Thiobrücke eintritt, z.B. gegenüber der bei höheren Temperaturen oftmals bei klassischen Polymeren beobachteten, oxidativen Zersetzung durch Radikalreaktionen.

Möglicherweise im Ausgangsmaterial vorhandene Reste R² werden bei der obigen Umsetzung der Verbindung mit der Formel (II) mit der Struktur (0) nicht angegriffen. Sie stehen daher im erhaltenen Silan oder Kieselsäure(hetero)polykondensat als Kopplungsgruppen zur Verfügung, die zu einer weiteren Modifizierung der Produkte eingesetzt werden können. An diese können bei Bedarf in einer zweiten Reaktionsstufe Verbindungen der Struktur (III) addiert werden.

In einer Ausführungsform der Erfindung lässt sich diese Möglichkeit nun dazu nutzen, die Anzahl an organisch polymerisierbaren C=C-Doppelbindungen (nichtaromatischen C=C-Doppelbindungen) der mit der Gruppierung -Y-(W)ₐ(Ar)_{b} versehenen Silane bzw. Kieselsäure(hetero)polykondensate nachträglich wieder zu erhöhen, um das organische Vernetzungspotenzial trotz des Verbrauchs an diesen Gruppen durch die erste Reaktion nochmals anzuheben. Dies gelingt durch die Umsetzung mit einer Verbindung der Formel (III) wobei die Umsetzung der nachstehenden Reaktionsgleichung gehorcht:

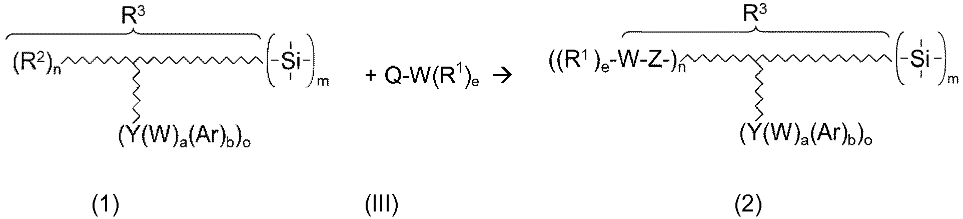

In der Verbindung der Formel (III) ist Q für alle Varianten auswählbar unter einer Isocyanatgruppe (OCN-) und einer Epoxygruppe. Außerdem kann Q für den Fall, dass R² eine OH-Gruppe ist, eine Carbonsäuregruppe -COOH sein, die ggf. in aktivierter Form vorliegt, während sie statt dessen für den Fall, dass R² eine Carbonsäuregruppe oder ein Salz oder ein Ester davon ist, außerdem eine OH-Gruppe sein kann. W besitzt die oben im Zusammenhang mit der Formel (II) angegebenen Bedeutung, R¹ ist eine geradkettige oder verzweigte, organisch polymerisierbaren Gruppe ist, die mindestens eine C=C-Doppelbindung trägt, und e bedeutet 1, 2, 3, 4 oder ein noch größere Zahl. Durch den Angriff des Restes Q an R² entsteht im Produkt eine Kupplungsgruppe Z. Im Falle des Angriffs einer Isocyanatgruppe an einer COOH-Gruppe bildet sich eine Säureamidgruppe -C(O)NH-, im Falle des Angriffs einer OH-Gruppe bildet sich eine Urethangruppe. Aus Q = COOH (bzw. einer aktivierten Form davon) und R² = OH bildet sich eine Estergruppe, genauso wie aus Q = OH und R² = COOH bzw. einem Ester oder Salz davon, die aber natürlich umgekehrt ausgerichtet ist. Dabei ist zu beachten, in der zuletzt aufgeführten Variante dann, wenn R² ein Carbonsäureester ist, eine Umesterung stattfindet. Um das Gleichgewicht auf die Seite des Produkts zu verschieben, sollte dabei die Alkoholkomponente dieses Esters (R* von R² = COOR*) vorzugsweise das Produkt eines primären und kleinen Alkohols R*OH sein. Wenn Q eine Epoxygruppe ist, bildet sich im Falle von R² = OH ein Ether (unter Ausbildung einer freien Hydroxygruppe), im Falle von R² = COOH ein Ester.

Z in der Verbindung bzw. Struktur (2) hat daher die Bedeutung einer Urethan-, Säureamid-, Ether- oder Estergruppe, wobei die Estergruppe beidseitig ausgerichtet sein kann. Über diese Kupplungsgruppe ist der Rest W-(R¹)ₑ an die Kohlenwasserstoffgruppe des Restes R³ angebunden.

Die voranstehend beschriebenen Reaktionen werden vorzugsweise an bereits teilweise oder völlig vorkondensierten Kieselsäure(hetero)polysiloxanen durchgeführt. Wie bereits zuvor erwähnt, ist es aber natürlich genauso gut möglich, erst die entsprechenden Silane herzustellen und diese anschließend in bekannter Weise einer hydrolytischen Kondensation zu unterwerfen. Unabhängig davon, welcher der beiden Wege beschritten wird, wird eine organische Polymerisationsreaktion wie oben beschrieben erst dann durchgeführt, wenn die Harze in ihre endgültige Form überführt wurden, beispielsweise in Form eines Restaurations-/ Zahnersatzmaterials im Mund eines Patienten, wo die organische Polymerisationsreaktion (Polyaddition) der Reste R¹ vorzugsweise über eine Lichthärtung induziert wird.

Die Umsetzung der Silane oder Strukturen (1) mit der Verbindung der Formel (III) kann stöchiometrisch, also äquimolar erfolgen, indem ein Mol Verbindung (III) (oder sogar ein Überschuss davon) pro Mol des Restes R² im Silan (1) oder in der Kieselsäurepolykondensat-Struktur (1) eingesetzt wird (im Falle eines Überschusses wird man diesen nach der Umsetzung wieder abtrennen). Dabei werden im Wesentlichen alle Reste R² in Reste (R¹)ₑ-W-Z überführt. Diese Umsetzung kann stattdessen jedoch ebenfalls unterstöchiometrisch erfolgen. Es entsteht dann entweder ein Gemisch an Silanen der Formeln (1) und (2) oder ein Kieselsäurepolykondensat, das Strukturen (1) neben Strukturen (2) aufweist.

In einer weiteren Ausführungsform der Erfindung, die entweder alternativ zur Umsetzung mit der Verbindung der Formel (III) durchgeführt oder mit der vorherigen kombiniert werden kann, wobei letzteres nur dann möglich ist, wenn bei der Umsetzung mit der Verbindung der Formel (III) nur ein Teil der Reste R² umgesetzt wurde, können die oder die verbliebenen Reste R² im Rahmen einer weiteren Reaktionsstufe zur Erzeugung eines zusätzlichen organischen Netzwerks genutzt werden, welches allerdings nicht aus polyaddierten C-C-Gruppierungen besteht. Dies wird durch die Umsetzung mit einer Verbindung (IV) ermöglicht, wie dem Grunde nach in DE 10 2005 018059 offenbart.

Bei dieser Umsetzung werden die Silane oder Strukturen der Formel (1), in denen mindestens ein Teil der Substituenten R³ Reste R² aufweisen (d.h. n > 0, vorzugsweise mindestens 0,2, stärker bevorzugt mindestens 0,5) umgesetzt mit einer Verbindung (IV), wobei die Umsetzung der nachstehenden Reaktionsgleichung gehorcht:

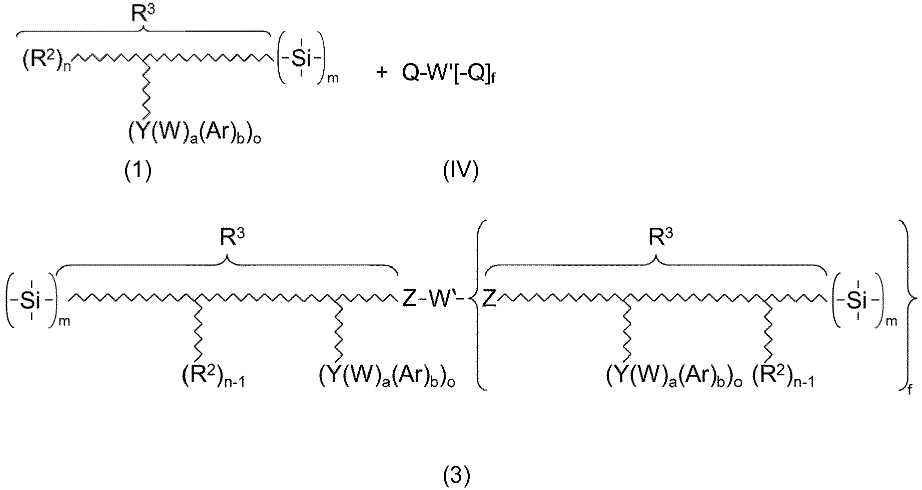

In der Verbindung (IV) ist Q ausgewählt unter-NCO, -OH, einer Epoxidgruppe oder-C(O)X', wobei -C(O)X' eine Carbonsäuregruppe oder eine aktivierte Carbonylverbindung, insbesondere eine Säurechlorid oder ein Säureanhydrid (mit X'= -O-C(O)-Alkyl, -Aryl oder -Alkylaryl/ Arylalkyl)) ist, bedeuten und Q -NCO oder -C(O)X' sein kann, wenn in der Struktur der Formel (1) R² OH bedeutet und worin Q -NCO oder -OH- sein kann, wenn in der Struktur der Formel (1) R² COOR mit R = OH oder ein salzbildendes Kation oder eine Estergruppe bedeutet. Q kann auch Teil eines cyclischen, intramolekularen Anhydrids sein; in diesen Fällen bilden 2 Reste Q eine Gruppe -C(O)O(O)C-, die an zwei Kohlenstoffatome von W' gebunden ist. W' kann in Ausnahmefällen (nämlich nur dann, wenn Q jeweils aktivierte Carbonsäuregruppen sind und f gleich 1 ist, d.h. nur in dem Falle, in dem die Verbindung mit der Formel (IV) Oxalsäure oder ein aktiviertes Oxalsäurederivat ist) eine Einfachbindung sein und ist im Übrigen ein "Spacer", d.h. ein beliebiger Rest mit f+1 Valenzen. Vorzugsweise besitzt oder ist W' eine ggf. durch Sauerstoffatome, Schwefelatome, Carbonylgruppen, Carboxylgruppen, Aminogruppen oder Amidgruppen unterbrochene Kohlenstoffkette, insbesondere eine Alkylenkette von 1 bis 60, stärker bevorzugt 1 bis 15 Kohlenstoffatomen. Stattdessen kann W' einen oder mehrere, ggf. (eine) weitere solche Kohlenstoffkette(n) aufweisende Ringe (Cycloalkylring, aromatischer Ring, mit oder ohne Heteroatome) enthalten oder eine reine Ringstruktur aufweisen, z.B. eine Phenylengruppe, eine Naphthylengruppe oder eine Diphenylengruppe sein. Sofern die Verbindung (IV) eine oder mehrere aromatische Gruppen aufweist, ist damit eine weitere Brechzahlanhebung möglich, weshalb diese Ausführungsform besonders bevorzugt ist. Die Ringe bzw. die Kohlenstoffketten können weiterhin ggf. substituiert sein, sofern ihre Substituenten nicht mit der Reaktion zwischen Q und R² interferieren. Ein Beispiel hierfür ist eine Cyano- oder Tricyanogruppe. f ist bevorzugt 1, kann aber auch 2, 3, 4, 5 oder 6 sein. Z.B. sind Di- Tri- oder Tetra-Anhydride, -Carbonsäuren (ggf. aktiviert bzw. aktivierbar), -Isocyanate oder -Alkohole in großem Umfang käuflich. Auch natürliche Oligomere können für die vorliegende Erfindung eingesetzt werden wie Zuckermoleküle. In Ausnahmefällen kann f sogar eine höhere Zahl als 6 bedeuten.

Durch den Angriff mehrerer Reste Q an mehreren Resten R² entsteht ein Produkt, bei dem über zwei Kupplungsgruppen Z und den "Spacer" W' mindestens zwei Silane bzw. Strukturen der Formel (1) miteinander verknüpft werden. Im Falle des Angriffs einer Isocyanatgruppe an einer COOH-Gruppe bildet sich eine Säureamidgruppe -C(O)NH-, im Falle des Angriffs einer OH-Gruppe bildet sich eine Urethangruppe. Aus Q = COOH (bzw. einer aktivierten Form davon) und R² = OH bildet sich eine Estergruppe, genauso wie aus Q = OH und R² = COOH bzw. einem Ester oder Salz davon, die aber natürlich umgekehrt ausgerichtet ist. Dabei ist zu beachten, in der zuletzt aufgeführten Variante dann, wenn R² ein Carbonsäureester ist, eine Umesterung stattfindet. Um das Gleichgewicht auf die Seite des Produkts zu verschieben, sollte dabei die Alkoholkomponente dieses Esters (R* von R² = COOR*) vorzugsweise das Produkt eines primären und kleinen Alkohols R*OH sein. Wenn Q eine Epoxygruppe ist, bildet sich im Falle von R² = OH ein Ether (unter Ausbildung eine benachbarten OH-Gruppe), im Falle von R² = COOH ein Ester.

Z in der Verbindung bzw. Struktur (3) hat daher die Bedeutung einer Urethan-, Säureamid-, Ether- oder Estergruppe, wobei die Estergruppe beidseitig ausgerichtet sein kann.

Die Verbindung mit der Formel (IV) kann spiegelsymmetrisch sein, muss es aber nicht.

Man erkennt leicht, dass sich die Umsetzungen mit den Verbindungen (III) bzw. (IV) nur dadurch unterscheiden, dass die Verbindung (IV) mehrere Reste Q aufweist und daher zur Verknüpfung mehrerer Silane oder Strukturen mit Substituenten bzw. Resten (3) geeignet ist, während die Verbindung (III) über dieselbe Anbindungsreaktion den Rest R¹ in den Substituenten oder Rest R³ einführt und damit eine Vernetzung über eine Polyadditionsreaktion (Polymerisationsreaktion im Sinne der englischen "chain growth polymeriziation" oder "addition polymerization") von C=C-Doppelbindungen ermöglicht, wie aus dem Stand der Technik bekannt. Daraus wird auch offensichtlich, dass je nach Bedarf entweder die eine oder die andere Umsetzung gewählt werden kann, oder aber eine Kombination beider Umsetzungen, wobei jeweils ein entsprechender Unterschuss zumindest der Verbindung (III) oder (IV) eingesetzt werden muss, die zuerst mit dem Silan oder der Struktur (1) umgesetzt werden soll. Durch die Wahl entsprechender Mengenverhältnisse (Äquivalente) lässt sich auch in diesem Punkt eine sehr feine Abstimmung der Produkteigenschaften erzielen. Dabei gilt allgemein die Regel: Wenn der Anteil an Verbindung (IV) relativ hoch gewählt wird, kann sich das zuvor flüssige Harz verfestigen, was nicht immer wünschenswert ist, weshalb in einer Reihe von Fällen beispielsweise ein Anteil von ≤ 0,2 Mol pro Rest R² günstig sein kann, und zwar vor allem dann, wenn bei der Reaktion mit der Verbindung (II) noch ein Großteil der doppelbindungshaltigen Reste R¹ verblieben ist und/oder eine Wiederherstellung solcher Reste über die Umsetzung mit der Verbindung (III) stattgefunden hat/gleichzeitig stattfindet, so dass eine spätere Verfestigung immer noch möglich ist. Wenn aber der Anteil an Resten R¹ aus dem einen und/oder dem anderen Grund niedrig ist oder gar keine Reste R¹ zur Verfügung stehen, das Harz also nicht durch eine Polymerisationsreaktion der C=C-Doppelbindungen dieser Reste nachgehärtet werden kann, kann es durchaus wünschenswert sein, den Anteil an Verbindung (IV) hoch zu wählen, um auf diesem Weg eine Vernetzung und damit Härtung des Kieselsäure(hetero)polykondensats herbeizuführen. Damit ist generell bei hohen Anteilen der Verbindung (IV) (z.B. annähernd oder genau (c+1) Mol pro Mol Silan mit einem Substituenten R³ oder pro Substituent R³ im Kieselsäure(hetero)polykondensat) auf diesem Wege eine auf rein organischem Wege erfolgende Nachhärtung der Harzsysteme möglich, ohne dass für diesen Zweck ein Teil der Reste R¹ unumgesetzt bleiben oder Reste R¹ über die Reaktion mit der Verbindung (III) wieder eingeführt werden müssten.

Eine spezielle Form der Nachhärtung nutzt nicht (oder nicht nur) die Polymerisationsreaktion (Polyaddition) der C=C-Doppelbindungen als solcher wie oben erläutert. Möglich ist nämlich auch eine Umsetzung der diese Doppelbindungen enthaltenden Silane bzw. Kieselsäurepolykondensate mit Di- oder höheren Aminen oder Di- oder höheren Thiolen über eine Michael-Addition (Thiol-En-Umsetzung bzw. die analoge Umsetzung mit Aminen). Geeignet hierfür sind Di-, Tri, Tetra- oder sogar noch höher funktionalisierte Amine oder Mercaptane, wobei die Reaktion mit Aminen (nur) dann gelingt, wenn die C=C-Doppelbindungen in aktivierter Form vorliegen, beispielsweise als Acryl- oder Methacrylgruppen (darunter (Meth)Acrylatgruppen). Hierdurch kann an die Stelle der Vernetzung durch lichtinduzierte organische Polyaddition ("chain growth polymerization") wie oben erwähnt die Vernetzung durch die genannten, mehrfach funktionellen Mercaptane bzw. Amine treten. Beispiele für polyfunktionelle Thiole sind: Trimethylolpropantri-(3-mercaptopropionat) (TMPMP); Trimethylolpropan-trimercaptoacetat) (TMPMA); Pentaerytritoltetra(3-mercaptopropionat) (PETMP); Pentaerytritoltetramercaptoacetat) (PETMA); Glykoldimercaptoacetat; Glykoldi(3-mercapto-propionat); Ethoxyliertes Trimethylolpropantri(3-mercaptopropionat); Biphenyl-4-4'-dithiol; p-Terphenyl-4,4"-dithiol; 4,4'-Thiobisbezenthiol; 4,4'-Dimercaptostilben; Benzen-1,3-dithiol; Benzen-1,2-dithiol; Benzen-1,4-dithiol; 1,2-Benzendimethanthiol; 1,3-Benzendimethanthiol; 1,4-Benzendimethanthiol; 2,2'-(Ethylendioxy)diethanthiol; 1,6-Hexandithiol; 1,8-Octandi-thiol; 1,9-Nonandithiol. Gleiches gelingt, wenn statt der oben beschriebenen Thiole Amine verwendet werden, sofern, wie erwähnt, die C=C-Doppelbindungen in aktivierter Form vorliegen. Beispiele für multifunktionelle Amine sind: Diaminoaceton, Diaminoacridin, Diaminoadamantan, Diaminoantrachinon, Benzidin, Diaminobenzoesäure, Phenylendiamin, Diaminobenzo-phenon, Diaminobutan, Diaminocyclohexan, Diaminodecan, Diaminodicyclohexylmethan, Diaminomethoxybiphenyl, Diaminodimethylhexan, Diaminodiphenylmethan, Diamino-dodecan, Diaminoheptan, Diaminomesitylen, Diaminomethylpentan, Diaminomethylpropan, Naphtyhlendiamin, Diaminoneopentan, Diaminooctan, Diaminopentan, Diaminophenantren, Diaminopropan, Diaminopropanol, Diaminopurin, Diaminopyrimidin. In der Regel wird die Thiol-Addition in Gegenwart eines Initiators durchgeführt, wie aus dem Stand der Technik bekannt, während die Amin-Addition auch ohne Initiator möglich ist.

Die genannte Nachhärtung (Polyaddition) kann an die Stelle der Polymerisationsreaktion der C=C-Doppelbindungen (der "chain growth polymerization") treten; es bildet sich dabei ein etwas lockereres organisches Netzwerk, weil S-(Kohlenwasserstoff)-S-Brücken bzw. N-(Kohlenwasserstoff)-N-Brücken ausgebildet werden. Sie kann aber auch zusätzlich erfolgen, indem die Menge an Di- bzw. Polythiolen oder Di- bzw. Polyaminen so gewählt wird, dass C=C-Doppelbindungen im Harz verbleiben, die anschließend auf üblichem Wege nachgehärtet werden können.

Bei der Vernetzung können weiterhin Eigenschaften wie die Länge der Molekülketten zwischen den Vernetzungsstellen sowie die verbleibenden Anteile an freien reaktiven Gruppen eingestellt werden, beispielsweise durch den Zusatz von variablen Anteilen an Di-, Tri- und/oder Tetra-Isocyanaten, die mit jeweils freien Gruppen Q reagieren können. Durch den Einsatz bis-, tris- oder noch höher funktioneller Verbindungen als Reaktionspartner mit der Formel (IV) werden Verknüpfungen über Brücken mit wahlweise einstellbarer Länge erzeugt (die Längeneinstellung erfolgt durch den Abstand der reaktiven Gruppen Q im Molekül). Als Beispiel hierfür diene die Umsetzung mit Isocyanaten: Hier können z.B. Dicyclohexylmethandiisocyanat, Hexamethylen-1,6-diisocyanat, Hexamethylen-1,8-diisocyanat, Diphenylmethan-4,4-diisocyanat, Diphenylmethan-2,4-diisocyanat, 2,4-Toluylendiisocyanat, 2,6-Toluylendiisocyanat, Triphenylmethan-4,4',4"-triisocyanat, 3-Isocyanatomethyl-3,5,5-trimethylcyclohexyl-diisocyanat, oder Tris(p-isocyanatophenyl)thiophosphat eingesetzt werden. Sofern es sich bei den Resten R² um eine Hydroxygruppe handelt, kann eine derartige Vernetzung z.B. auch mit einer di-, tri-, tetra oder polyfunktionellen, ggf. aktivierten (z.B. in Form eines Anhydrids vorliegenden) Carbonsäure anstelle des Di-, Tri- oder Tetrapolyisocyanats erfolgen, während dann, wenn es sich bei diesem Rest um einen freien Carbonsäurerest, oder dessen Salz oder Ester handelt, aufweist, die Vernetzung statt dessen auch mit einem di-, tri-, tetra oder polyfunktionellen Alkohol erfolgen kann.

Die erfindungsgemäßen Harze werden für dentale Zwecke im Allgemeinen in mit Partikeln gefüllter Form eingesetzt. Geeignete Füllstoffe sind beispielsweise diejenigen, die in der DE 196 43781, DE 19832965, DE 10018405, DE 1041038, DE102005018351, DE102005061965.7 und insbesondere in der DE102011054444.5 beschrieben sind. Bei diesen Füllstoffen handelt es sich um Beispiele für prinzipiell mögliche Füllstoffe zur Kompositherstellung. Bevorzugt ist allerdings, dass klassische, kommerziell erhältliche Dentalglasfüller z. B. der Fa. Schott, zumindest einen wesentlich Anteil des Füllstoffs (vorzugsweise über 50 Gew.-%, stärker bevorzugt über 75 Gew.-%) bilden. In diesen Ausführungsformen kann der Rest z.B. aus Füllstoffen, wie sie in den oben genannten Druckschriften genannt sind, bestehen. Durch Rückgriff auf die bevorzugten Dentalglasfüller, ggf. in Kombination mit weiteren Füllstoffen wie voranstehend dargestellt, ist es möglich, die Brechzahl der erfindungsgemäßen Harzsysteme und diejenige des Füllstoffs/Dentalglases soweit aneinander anzupassen, dass sie fast (oder, vorzugsweise, sogar vollständig) identisch sind. Es lassen sich Brechzahldifferenzen von Δ_{nD} im Bereich von ± 0,001, häufig von ± 0,0005 erreichen. Auf diesem Wege erhält man die gewünschte, auch notwendige Transluzenz der erfindungsgemäßen Komposite, die sich damit als hochästhetische dentale Werkstoffe einsetzen lassen.

Sofern die voranstehenden Umsetzungen an Silanverbindungen erfolgen, können diese, wie bereits erwähnt, anschließend einer hydrolytischen Kondensation unterworfen werden. Eine derartige Kondensationsreaktion kann entweder an einer einzigen erfindungsgemäßen Silanverbindung oder an einer Mischung aus mehreren Silanverbindungen, die durch die erfindungsgemäße(n) Umsetzung(en) mit einem Unterschuss an der jeweiligen Verbindung (II), (III) oder (IV) entstehen. In beiden Fällen können je nach Bedarf weitere, bekannte Silane oder Alkoxymetallverbindungen wie Alkoxide des Aluminiums, Titans, Bors oder Zirkoniums oder von weiteren Übergangsmetallen einkondensiert werden. Durch den Zusatz von Silanen mit vier hydrolysierbaren Gruppen wie Tetraalkoxysilanen und/oder Alkoxymetallverbindungen kann beispielsweise das anorganische Netzwerk gefestigt und die Anzahl der organischen Substituenten "verdünnt" werden, während man durch den Zusatz von Silanen mit zwei oder drei derartigen hydrolysierbaren Gruppen und einem oder zwei über Kohlenstoff an Silicium gebundenen Substituenten verschiedenartige Modifikationen vornehmen kann. So modifiziert eine höhere Anzahl von silangebundenen Alkylgruppen die mechanischen Eigenschaften des letztendlich ausgehärteten Kieselsäurepolykondensats, während silangebundene Kohlenwasserstoffgruppen mit reaktiven Substituenten die Möglichkeit zusätzlicher Reaktionen ermöglichen. Silangebundene Kohlenwasserstoffgruppen, die organisch polymerisierbare C=C-Doppelbindungen enthalten, können wiederum zugesetzt werden, um die organische Vernetzungsmöglichkeit für den Fall zu gewährleisten, dass unter spezifischen Umständen die Umsetzungen des Silans bzw. der Struktur (1) mit Verbindungen der Formel (II) und/oder (III) hierfür entweder nicht ausreichen oder aus anderen Gründen nicht durchgeführt werden sollen.

In allen Fällen ist es bevorzugt, die hydrolytische Kondensation der Silane durchzuführen, bevor die organischen Vernetzungsreaktionen durchgeführt werden, sei es durch die Polymerisation der Reste R¹, sei es durch die Umsetzung der Verbindungen (1) mit einer Verbindung (IV). Der umgekehrte Weg ist jedoch nicht ausgeschlossen; er kann unter spezifischen Umständen der geeignetere sein.

Mit den in der vorliegenden Anmeldung beschriebenen neuartigen funktionalisierten Harz-/ Matrix-systemen stehen Materialien zur Verfügung, die einerseits aufgrund ihrer besonderen Eigenschaften (feinjustierbare Brechzahl bei anpassbarem E-Modul, ggf. matrixinterne Stabilisierung durch Thiobrücken) direkt verwendet werden können und sich daher z.B. aufgrund der durch die Einführung der Gruppierungen -Y-(W)ₐ(Ar)_{b} erzielten Brechzahlsteigerung für besondere optische Anwendungen eignen können, andererseits aber gerade aufgrund der feinen Justierbarkeit der Brechzahl mit Füllstoffen andererseits zu beispielsweise im dentalen Bereich anwendbaren Kompositen verarbeiten lassen, bei denen je nach Bedarf die Brechzahlen des Harzes und des Füllstoffes annähernd oder ganz identisch gewählt werden können, so dass eine hohe Transluzenz erreicht wird. Die Harze zeichnen sich durch die Möglichkeit aus, plastisch verarbeitbare Komposite mit sehr hohen Füllstoffgehalten (bis über 85 Masse-%, in Einzelfällen sogar bis über 89 Masse%, und damit über 80 Vol.-%) zu erzeugen. In gehärteter Form zeigen sie einen an die jeweilige Anwendung anpassbaren E-Modul bei hoher Festigkeit, eine hohe Abrasionsresistenz, eine sehr geringe Schrumpfung, eine ausgezeichnete Ästhetik (anpassbare Transluzenz bis zu vollständiger Transluzenz), Monomerfreiheit und damit eine hohe Biokompatibilität. Durch die z. T. festgestellte matrixinterne Stabilisierung (Vermeidung/Reduktion der oxidativen Zersetzung von -S-Gruppen enthaltenden Harzen durch Radikalreaktionen) ist ebenfalls ein Einsatz bei erhöhten Temperaturen (z.B. bis auf ca. 100°C) möglich.

Der Einsatz der erfindungsgemäßen Materialien ist vielgestaltig; er erstreckt sich zum Beispiel für die verschiedensten Zwecke auf die Verwendung in Form von Bulkmaterialien, Kompositen, Zementen, Klebstoffen, Vergussmassen, Beschichtungsmaterialien, Haftvermittlern, zur Herstellung bzw. Primung von Füllstoffen und Fasern, und zur Verarbeitung im (Reaktions)Extruder. Insbesondere ist ein Einsatz für (zahn-)medizinische und für (mikro)optische und (mikro)elektronische Anwendungen von Bedeutung.

Besonders bevorzugt ist der Einsatz im
▪ Dentalbereich, z. B. als Restaurations-/Zahnersatz (Kronen, Brücken, Inlays, Onlays, Veneers, künstliche Zähne, Implantataufbauten, Abutments,) oder Prophylaxematerial, Adhäsiv, Ionomerzement, in der Prothetik, als Implantatmaterial,
▪ Bereich der Mehrphotonenpolymerisation (sofern das Material Reste R¹ aufweist): Das flüssige Harz kann in einem Bad mit fokussierten Laserstrahlen behandelt werden, derart, dass nur im Fokus der Laserstrahlung eine Verfestigung durch die Polymerisation der C=C-Gruppen in den Resten R¹ auftritt, wodurch sich innerhalb des Bades ein Festkörper mit beliebigen Formen und genauen Abmessungen erzeugen lässt. Dieser Einsatz ist dem Bereich der Optik zuzuordnen.

Die vorliegende Erfindung besitzt weiterhin die folgenden Vorteile:
▪ Es handelt sich bei den beschriebenen Additionsreaktionen in der Regel um sehr schnelle und einfache Umsetzungen unter milden Bedingungen (z. T. ohne Katalysator bzw. mit sehr geringer Katalysator-Konzentrationen) ohne zusätzliche Aufarbeitungs-/ Reinigungsschritte, die zu den höher brechenden Strukturen (Umsetzung zur Verbindung bzw. Struktur (1)), den Strukturen mit erhöhtem Vernetzungspotenzial bei erhöhter Brechzahl (Umsetzung der Verbindungen bzw. Strukturen (1) mit einer Verbindung (III)) bzw. den vernetzten Strukturen mit ggf. weiter erhöhter Brechzahl (Umsetzung der Verbindungen bzw. Strukturen (1) mit deiner Verbindung der Formel (IV)) führen. Dazu Folgendes: die Umsetzung der Verbindungen bzw. Strukturen der Formel (0) mit der Verbindung (II), d.h. eine HS- oder HNR-Addition an eine C=C-Doppelbindung, erfolgt zumindest in den bevorzugten Fällen, in denen diese Gruppe z.B. durch ihre Einbindung in eine (Meth)Acryl-Struktur aktiviert ist, ohne Katalysator. Dem Fachmann sind die im Übrigen erforderlichen Maßnahmen bekannt.
▪ Die Erfindung ermöglicht ausgehend von bereits existierenden Silanen und Harzsystemen durch deren nahezu stufenlose Modifikationsmöglichkeiten wie voranstehend dargestellt, insbesondere infolge der feinen Justierbarkeit von Brechzahl und E-Modul wie oben erwähnt eine Brechzahlanpassung an z. B. beliebige kommerziell erhältliche Füllstoffe, weshalb die Notwendigkeit, zur Erzeugung einer gewünschten, meist extrem hohen Transluzenz auf besonders angepasste und damit teure Füllstoffe zurückgreifen zu müssen, wegfällt. Damit ist einerseits im Dentalbereich die geforderte Ästhetik von dentalen direkten/indirekten Restaurations-/Zahnersatzmaterialien realisierbar, während andererseits das Erzielen einer vollständigen Transparenz optische Anwendungen ermöglicht. Darüber hinaus lassen sich, wie oben erwähnt, die mechanischen Eigenschaften, z.B. der E-Modul unabhängig von den optischen Eigenschaften auf die jeweils erforderlichen Werte einstellen.
▪ Für die Anwendung von Materialien im Dentalbereich ist es wichtig, dass diese keine allergischen Reaktionen auslösen können. Es ist bekannt, dass einige (meth)acrylat-basierte Monomere aus toxokologischer Sicht problematisch sind, weshalb für die genannten Anwendungen in der Regel auf diese verzichtet werden muss. Sofern jedoch die Materialien der vorliegenden Erfindung (Meth)Acrylgruppen aufweisen, beispielsweise in Form der Reste R¹ oder als Bestandteil von Resten R¹, besteht keinerlei Gefährdung, da sie im Harz in jedem Fall mit der anorganischen Polymerstruktur (basierend auf der anorganischen Vernetzung über Si-O-Si-Brücken) verbunden sind und somit weder im nicht ausgehärteten Zustand (in dem die Harze möglicherweise dental appliziert werden) noch nach der Härtung (z.B. durch lichtinduzierte Vernetzung der C=C-Doppelbindungen im Mund des Patienten) in monomerer Form vorhanden sein und aus dem Dentalkörper austreten können. Im Übrigen sei darauf hingewiesen, dass diese Überlegungen keineswegs ausschließen, dass den erfindungsgemäßen Silanen, Kieselsäure(hetero)polykondensaten und Kompositen bei Bedarf organische Monomere zugesetzt werden können.
▪ Von besonderer Bedeutung ist die sehr einfache, nahezu stufenlose Modifikationsmöglichkeit und damit feine Justierbarkeit der erfindungsgemäßen Kieselsäure(hetero)polykondensate bzw. den daraus erzeugbaren Kompositen, wobei die Erfindung von bereits im Stand der Technik vorhandenen Harzsystemen (ggf. statt dessen Silanen) ausgeht und dem Fachmann eine Auswahl geeigneter Materialien aus dieser Gruppe an die Hand gibt. Die erste bzw. einzige Umsetzung erfolgt mit einem meist chemisch einfach aufgebauten und daher in der Regel kostengünstigen Reaktionspartner; die Reaktionsbedingungen können milde gewählt werden, und zusätzliche Aufarbeitungs- oder Reinigungsschritte sind nur in Ausnahmefällen erforderlich.

Nachfolgend soll die Erfindung anhand von Ausführungsbeispielen näher erläutert werden.

### Ausführungsbeispiele

### Beispiel Ia. Synthese von Basisharzsystem-1 mit einer Kopplungsgruppe R² (= OH) (Herstellung gemäß DE 4416857)

Zur Vorlage von 125,0 g (0,503 mol) 3-Glycidyloxypropylmethyldimethoxysilan (MG = 236) werden unter trockener Atmosphäre Triphenylphosphin als Kat., BHT als Stabilisator und anschließend 47,35 g (0,550 mol) Methacrylsäure zugetropft und bei 80°C gerührt (ca. 24 h).

Die Umsetzung kann über die Abnahme der Carbonsäurekonzentration mittels Säuretitration sowie dem Epoxidumsatz mittels Ramanspektroskopie/Epoxidtitration verfolgt werden. Die für die Epoxidgruppe vom Epoxysilan charakteristische Bande erscheint im Ramanspektrum bei 1256 cm⁻¹. Nach Zugabe von Essigester (1000 ml/mol Silan) und H₂O zur Hydrolyse mit HCl als Kat. wird bei 30°C gerührt. Die Aufarbeitung erfolgt nach ca. mehrtägigem Rühren durch mehrmaliges Ausschütteln mit wässriger NaOH und mit Wasser und Filtration über hydrophobierten Filter. Es wird zunächst abrotiert und anschließend mit Ölpumpenvakuum abgezogen. Es resultiert ein flüssiges Harz ohne Einsatz von Reaktivverdünnern (Monomeren) mit einer sehr geringen Viskosität von ca. 3 - 5) Pa·s bei 25 °C und 0,00 mmol CO₂H/g (keine freien Carboxylgruppen) sowie einer Brechzahl n_{D} von ca. 1,477.

### Beispiel Ib. Synthese von Basisharzsystem-2 ohne eine Kopplungsgruppe R² (Herstellung gemäß DE 4011044)

Zur Vorlage von 178 g (0,60 mol) Trimethylolpropantriacrylat (TMPTA), gelöst in Essigester (1 l/mol TMPTA), werden unter Argon und Rühren schnell 90,2 g (0,50 mol) Mercaptopropylmethyldimethoxysilan sowie eine ethanolische KOH mit 5,0 mmol KOH zugetropft (unter Kühlung, so dass die Temp. unter 30°C bleibt). Das R.-Gemisch wird einige Minuten bei RT weiter gerührt. Die Vollständigkeit der Umsetzung (Thioladdition) kann mittels Jod-Mercaptan-Test überprüft werden. Nach Zugabe von H₂O zur Hydrolyse/Kondensation mit HCl als Kat. wird bei RT gerührt. Die Aufarbeitung erfolgt nach ca. mehrtägigem Rühren durch mehrmaliges Ausschütteln mit Wasser und Filtration über hydrophobierten Filter. Es wird zunächst abrotiert und anschließend mit Ölpumpenvakuum abgezogen. Es resultiert ein flüssiges Harz.

### Beispiel IIa. Synthese der Beispielreihe 1 - 1. Reaktionsstufe mit Kopplungsgruppe R² (= OH)

### Grundreaktionsprinzip:

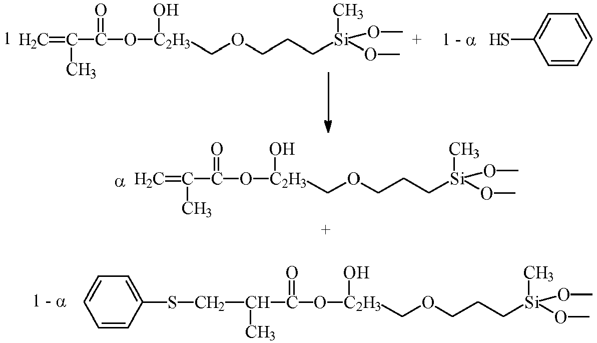

### Beispiel IIa-1 (1 - α = 0,2):

Zur Vorlage von 26,8 g (0,10 mol) von Basisharzsystem-1 werden unter Rühren 2,20 g (0,020 mol) Thiophenol zugetropft. Die Umsetzung kann z. B. mittels NMR verfolgt werden. Es resultiert ein flüssiges Harz mit einer Viskosität von ca. 5,8 - 6,0 Pa·s bei 25°C und einer Brechzahl n_{D} von ca. 1,493. Eine weitere Aufarbeitung ist nicht erforderlich.

### Beispiel IIa-2 (1 - α = 0,4):

Zur Vorlage von 39,7 g (0,15 mol) von Basisharzsystem-1 werden unter Rühren 6,61 g (0,079 mol) Thiophenol zugetropft. Es resultiert ein flüssiges Harz mit einer Viskosität von ca. 7,0 - 7,2 Pa·s bei 25°C und einer Brechzahl n_{D} von ca. 1,506. Eine weitere Aufarbeitung ist nicht erforderlich.

### Beispiel IIa-3 (1 - α = 0,45):

Zur Vorlage von 119,7 g (0,45 mol) von Basisharzsystem-1 werden unter Rühren 22,3 g (0,203 mol) Thiophenol zugetropft. Die Umsetzung kann z. B. mittels NMR verfolgt werden. Es resultiert ein flüssiges Harz mit einer Viskosität von ca. 7 Pa·s bei 25°C und einer Brechzahl n_{D} von ca. 1,508. Eine weitere Aufarbeitung ist nicht erforderlich.

Anzumerken ist, dass die Brechzahl gegenüber dem Produkt des Beispiels IIa-2 durch den leicht erhöhten Thiophenol-Anteil nur noch leicht (um 0,002) angestiegen ist. Sie lässt sich demnach sehr fein justieren.

Aus einem Vergleich der Beispiele la (Vergleich; Ausgangsmaterial), 1a und 1b lässt sich entnehmen, dass die Brechzahl über den Thiophenol-Anteil fein einstellbar ist: Man erkennt bei Einsatz von 20 Mol-% Thiophenol als Verbindung (II) einen leichter Anstieg gegenüber dem Basisharzsystem-1, der bei einer Verdoppelung des molaren Anteils der Verbindung (II) weiter steigt.

In allen drei Beispielen entsteht ein flüssiges Harz. Dessen Viskosität steigt von Beispiel IIa-1 bis IIa-3 nur geringfügig an.

### Beispiel IIb. Synthese der Beispielreihe 2 - 1. Reaktionsstufe mit Kopplungsgruppe R² (= OH)

### Grundreaktionsprinzip:

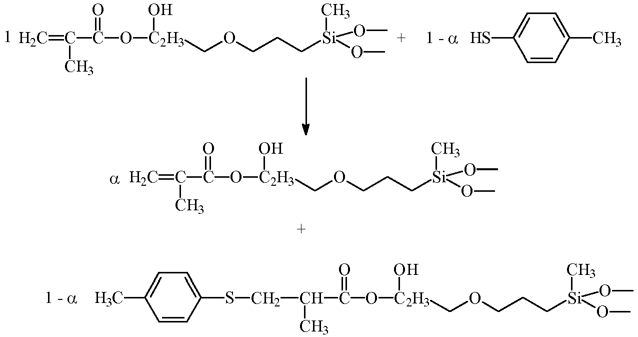

Für 1-α = 0,4 werden die folgenden Bestandteile eingesetzt: Zur Vorlage von 2,65 g (0,01 mol) von Basisharzsystem-1 werden unter Rühren 0,50 g (0,0044 mol) Methylbenzenthiol zugetropft. Die Umsetzung kann z. B. mittels NMR verfolgt werden. Es resultiert ein flüssiges Harz bei 25 °C und einer Brechzahl n_{D} von ca. 1,506). Eine weitere Aufarbeitung ist in der Regel nicht erforderlich.

### Beispiel IIc. Synthese der Beispielreihe 5 - 1. Reaktionsstufe (mit Kopplungsgruppe R² (= OH)

### Grundreaktionsprinzip:

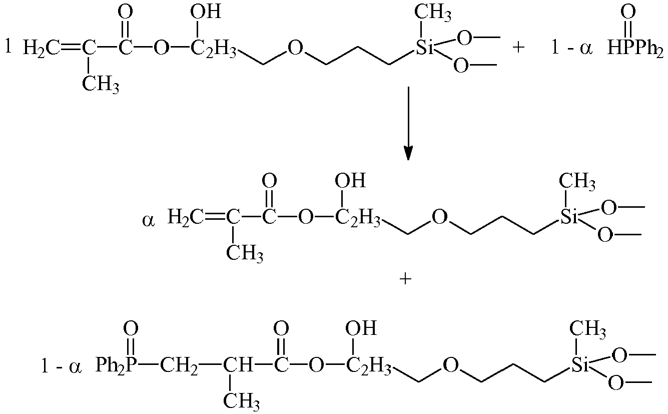

### Beispiel IIc-1 (1 - α = 0,2):

Zur Vorlage von 16,0 g (0,0615 mol) von Basisharzsystem-1 werden unter Rühren 2,48 g (0,0123 mol) Diphenylphosphinoxid und 0,2 ml 1,8-Diazabicyclo[5.4.0]undec-7-en (DBU) als Katalysator zugegeben. Das resultierende Reaktionsgemisch wird bei ≈ 50 °C bis zur vollständigen Addition gerührt. Die Umsetzung kann z. B. mittels NMR verfolgt werden. Nach üblicher Aufarbeitung z. B. in Essigester (ausschütteln mit Wasser, Filtration über hydrophobierten Filter, abrotieren und abschließendes Abziehen mit Ölpumpenvakuum) resultiert ein flüssiges Harz mit einer Viskosität von ca. 37 Pa·s bei 25 °C und einer Brechzahl n_{D} ≈ 1,500. Die Brechzahl des Produkts dieses Beispiels ist gegenüber der des Beispiels IIa-1 (n_{D} ≈ 1,493, Umsetzung von Basisharzsystem-1 mit 0,2 Molanteilen HS-Ph; siehe die Beispielreihe 1) bei ebenfalls 0,2 Molanteil des Addukts HP(O)(Ph)₂ erhöht, d.h. die Brechzahl lässt sich auf einem höheren Niveau einstellen. Dies beruht auf der Anwesenheit von zwei Phenylgruppen pro addiertem Molekül. Die Kopplungsgruppe R² = OH wird bei dieser Reaktion nicht angegriffen und kann daher zur weiteren Addition von C=C-haltigen Verbindungen (z. B. entsprechend Beispiel IIIa-1; siehe die Beispielreihe 4) genutzt werden.

### Beispiel IId. Synthese der Beispielreihe 3 - 1. Reaktionsstufe (ohne Kopplungsgruppe R²)

### Grundreaktionsprinzip:

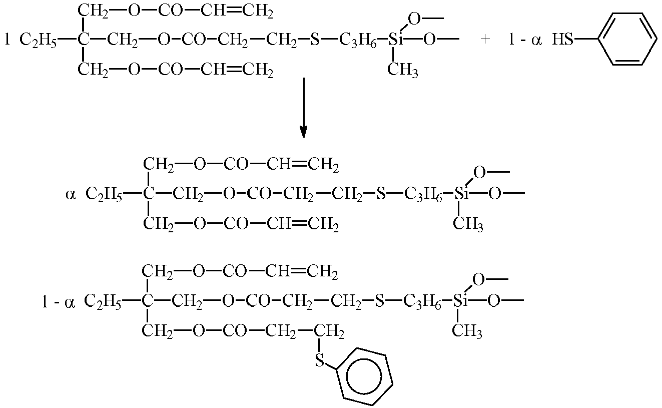

Das Basisharzsystem-2 (Produkt des Beispiels Ib) wurde mit verschiedenen Mengen an Thiophenol umgesetzt, wobei jedoch darauf geachtet wurde, dass die molare Menge (1-α) an Thiophenol unter derjenigen der Siliciumreste lag.

### Beispiel IIIa. Synthese der Beispielreihe 4 - 2. Reaktionsstufe (mit Kopplungsgruppe R² (= OH) und Y = -NCO)

### Grundreaktionsprinzip:

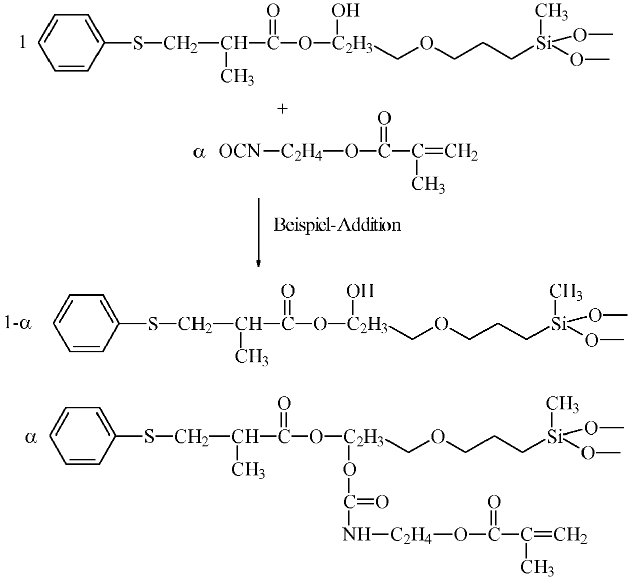

### Beispiel IIIa-1 (α = 0,8):

Zur Vorlage von 15,4 g aus Beispiel IIa-2 (0,05 mol) und 0,043 g BHT (2,6-Di-tert.-butyl-4-methylphenol) werden unter trockener Atmosphäre bei 30°C unter Rühren 6,21 g (0,04 mol) Methacrylsäure-isocyanatoethylester (0,8 Mol pro Mol siliciumgebundenem Rest R³) zugetropft und bei 30°C weitergerührt. Die Umsetzung kann z. B. über die Abnahme der OCN-Bande mittels IR-Spektrum verfolgt werden. Die für die OCN-Gruppe charakteristische Bande erscheint im IR-Spektrum bei ≈ 2272 cm⁻¹. Es resultiert ein flüssiges Harz mit einer Viskosität von ca. 36 Pa·s bei 25 °C und einer Brechzahl n_{D} von ca. 1,502. Eine weitere Aufarbeitung ist nicht erforderlich.

### Beispiel IIIa-2 (α = 0,8):

Zur Vorlage von 82,1 g aus Beispiel IIa-3 (0,26 mol) und 0,23 g BHT werden unter trockener Atmosphäre bei 30°C unter Rühren 32,3 g (0,208 mol) Methacrylsäureisocyanato-ethylester (0,8 Mol pro Mol siliciumgebundenem Rest R³) zugetropft und bei 30°C weitergerührt. Die Umsetzung kann z. B. über die Abnahme der OCN-Bande mittels IR-Spektrum verfolgt werden. Die für die OCN-Gruppe charakteristische Bande erscheint im IR-Spektrum bei ≈ 2272 cm⁻¹. Es resultiert ein flüssiges Harz mit einer Viskosität von ca. 37,6 Pa·s bei 25°C und einer Brechzahl n_{D} von ca. 1,5044.

Anzumerken ist, dass die Brechzahl gegenüber dem Produkt des Beispiels 4a durch den leicht erhöhten Thiophenol-Anteil leicht (um 0,0024) angestiegen ist.

### B. Kompositherstellung

### B1. Erfindungsgemäßes Komposit auf Basis des Harzes aus Beispiel IIIa-1

Ein Dentalglas der Fa. Schott mit der Bezeichnung GM27884 (n_{D} = 1,53) und der Spezifikation UF0.7 (Primärpartikelgröße von 0,7 µm) wird zunächst mittels 3-Walzwerk in das Harz des Beispiels IIIa-1 eingearbeitet. Anschließend wird ein solches der Spezifikation K6 (Primärpartikelgröße von 3 µm) mittels Planetenmischer eingearbeitet. Die Gläser wurden im Gewichtsverhältnis 1 : 2 (Glas der Spezifikation UF0.7 zu Glas der Spezifikation K6) zugegeben. Die Gesamtmenge an Glas im Komposit betrug 70 Gew.-%.

### B2. Erfindungsgemäßes Komposit auf Basis des Harzes aus Beispiel IIIa-1

Beispiel B1 wurde mit der Änderung wiederholt, dass als Dentalglas ein solches der Fa. Schott mit der Bezeichnung GM32087 (n_{D} = 1,52) und der Spezifikation UF0.4 (Primärpartikelgröße von 0,4 µm) sowie ein solches der Spezifikation K6 (Primärpartikelgröße von 3 µm) eingesetzt wurde.

### B3. Vergleichskomposit auf Basis von Basisharzsytem-1

Beispiel B2 wurde mit der Änderung wiederholt, dass anstelle des Harzes des Beispiels IIIa-1 das Basisharzsystem-1 als Komposit-Matrix diente.

### C. Mechanische und optische Daten nach Härtung

### Polymerisation/Härtung verschiedener Harzsysteme und Komposite im Vergleich zum zugrundeliegenden Basisharz

Die Harze aus den Beispielreihen bzw. des Basisharzsystems 1 und die daraus resultierenden Komposit wurden jeweils mit 1% Lucirin TPO in eine Stäbchenform (2 x 2 x 25 mm³) gegeben. Die (Meth)Acrylatgruppen werden im Rahmen einer photoinduzierten radikalischen Polymerisation umgesetzt, wobei das Harz aushärtet. Mittels 3-Punktbiegeversuch auf der Universalprüfmaschine Z100 der Zwick GmbH&Co. KG wird nach 1,5 Tagen Lagerung bei 40°C der E-Modul, die Bruchfestigkeit sowie die Durchbiegung bis zum Bruch der resultierenden Stäbchen bestimmt. Nach entsprechender Herstellung von plättchenförmigen Prüfkörper wird die Brechzahl mittels Refraktometer bestimmt. Die Schrumpfungswerte werden mittels Auftriebsmethode im Rahmen der photoinduzierten radikalischen Polymerisation erhalten (15 min /1 Tg nach Belichtung). Die Komposite mit 1% Lucirin TPO werden in eine Form (h = 2 mm; d = 18 mm) gegeben und im Rahmen einer photoinduzierten radikalischen Polymerisation ausgehärtet. Die Transluzenz wird mittels Spektrophotometer Color i7 von x-rite bestimmt.

**Tabelle 1**

| Harzsystem | Bruchfestigkeit [MPa] | E-Modul [GPa] | Durchbiegung [mm] | Brechzahl n_{D} | Schrumpfung [Vol.-%] 15 min / 1 Tg |
|---|---|---|---|---|---|
| **Basisharzsystem-1 (Vergleich)** | ≈ 89 | ≈ 1,94 | ≈ 3,2 | 1,504 | 5,2/5,8 |

| **1. Reaktionsstufe (mit Kopplungsgruppe R² (= OH)** | | | | | |
|---|---|---|---|---|---|
| IIa-1 | ≈ 71 | ≈ 1,43 | ≈ 3,3 | ≈ 1,519 | n.b. |
| IIa-2 | Keine realen Werte, da sehr hohe Durchbiegung | | | ≈ 1,525 | 4,0/4,1 |
| IIc-1 | ≈ 94 | ≈ 2,22 | ≈ 2,8 | 1,519 | |

| **2. Reaktionsstufe (mit Kopplungsgruppe R² (= OH) und Y = -NCO)** | | | | | |
|---|---|---|---|---|---|
| IIIa-1 | ≈ 110 - 116 | ≈ 2,36 - 2,42 | ≈ 2,54 - 2,71 | ≈ 1,524 | 4,7 / 5,7 |

| | | | | | |
|---|---|---|---|---|---|
| (Bei Mehrfachmessungen sind die in der Tabelle angegebenen Werte Mittelwerte, sofern kein Bereich angegeben ist) | | | | | |

Bei der Addition der Verbindung der Formel (II) in der 1. Reaktionsstufe wird neben dem Brechzahlanstieg nicht nur der erwartete E-Modulabfall sowie eine deutliche Reduktion der Schrumpfung, sondern überraschenderweise (insbesondere bei hohem Anteil an (II)) auch eine stark flexibilisierende Wirkung auf das gehärtete Material festgestellt.

Dagegen liegen die Festigkeit und der E-Modul (trotz des infolge der der Addition an einen Teil der vorhandenen Doppelbindungen reduzierten Doppelbindungsanteils) bei der Umsetzung IIc-1 sogar noch leicht oberhalb des zugrunde liegenden Basisharzsystem-1.

Die Brechzahl ist in allen Fällen der 1. Reaktionstufe signifikant erhöht.

Mit den gehärteten Materialien der 2. Reaktionsstufe (Addition einer Verbindung der Formel (III)) wird überraschenderweise auch bei hohem Anteil von vorher addierter Verbindung (II) (mit der Folge eines sehr geringen organischen Vernetzungspotenzials und infolgedessen einer flexibilisierende Wirkung und Reduktion der Festigkeit) wieder ein hartes, hochfestes Material mit erhöhter Brechzahl, mit im Vergleich zum zugrunde liegenden Basisharzsystem (hier Basisharzsystem-1) deutlich verbesserten mechanischen Daten und mit reduzierter Schrumpfung erhalten, während die Brechzahl signifikant erhöht ist.

**Tabelle 2**

| Komposit auf Basis von | Füllstoffe | | Bruchfestigkeit [MPa] | E-Modul [GPa] | Durchbiegung [mm] | Transluzenz [%] |
|---|---|---|---|---|---|---|
| | Gehalt [Gew.-%] | Typ / Verhältnis der Füllstoffe untereinander | | | | |
| B3 | 70 | GM27884 / UF0.7 : K6 = 1 : 2 | ≈ 131 | ≈ 6,95 | ≈ 0,73 | 38 |
| Nach 2. Reaktionsstufe (mit Kopplungsgruppe R² (= OH) und Y = -NCO) | | | | | | |
| B1 | 70 | GM27884 / UF0.7 : K6 = 1 : 2 | ≈ 151 | ≈ 7,92 | ≈ 0,71 | 61 |
| B2 | 70 | GM32087 / UF0.4 : K6 = 1 : 2 | ≈ 147 | ≈ 8,19 | ≈ 0,67 | 58 |

Die Brechzahl des gehärteten Harzsystems IIIa-1 liegt genau zwischen denen der beiden eingesetzten Füllstoffe. Mit beiden Kompositen (B1. und B2.) auf Basis des modifizierten Harzsystems IIIa-1 wird bei gleichem Füllstoffgehalt/Typ/Verhältnis eine signifikante Steigerung der Tranzluzenz bei gleichzeitiger Steigerung der Festigkeit und des E-Moduls im Vergleich zum Komposit auf Basis des unmodifizierten Basisharzsystems-1 (Vergleichsbeispiel B3.) erzielt. Damit werden hochtransluzente und gleichzeitig sehr feste Komposite zugänglich.

## Patentansprüche

1. Verbindung der Formel (1) oder Kieselsäure(hetero)polykondensat, umfassend hydrolytisch kondensierbare Strukturen der Formel (1), 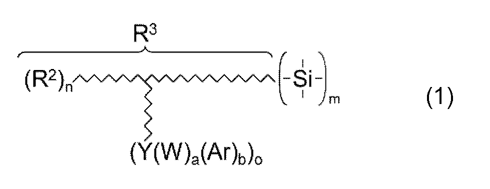 worin
R² eine Hydroxygruppe oder ein freier Carbonsäurerest oder ein davon abgeleiteter Carbonsäureester oder ein davon abgeleitetes Salz ist,
R³ einen Rest mit einem kohlenwasserstoffhaltigen, verzweigten oder unverzweigten Rückgrat bezeichnet, der über Kohlenstoff am Silicium gebunden ist und beliebig durch Heteroatome oder Kupplungsgruppen oder andere, Heteroatome enthaltende Gruppen unterbrochen sein darf, wobei die Zickzacklinie rein schematisch das kohlenwasserstoffhaltige Rückgrat bezeichnet,
die drei nicht näher gekennzeichneten Bindungen des Si-Atoms für weitere, am Siliciumatom gebundene Reste stehen, ausgewählt unter von Silicium abhydrolysierbaren Resten, Hydroxygruppen und über ein Kohlenstoffatom am Silicium gebundenen Resten, die dieselbe Bedeutung wie R³ oder eine davon abweichende andere Bedeutung haben können oder Sauerstoffbrücken zu weiteren Siliciumatomen und/oder anderen Metallatomen darstellen, wenn die Struktur (1) Bestandteil eines Kieselsäure(hetero)polykondensat ist,
der Rest Y entweder zweibindig ist und dann die Bedeutung -S-, -NR⁴- oder -P(O)(R⁴)_{c}(Z')_{d}- mit Z' = OR⁴, c = 0 oder 1, d = 0 oder 1 und (c+d) = 1 hat oder dreibindig ist und -N= oder -P(O)= bedeutet,
R⁴ einen kohlenwasserstoffhaltigen Rest darstellt und im Rest -NR⁴- darüber hinaus außerdem Wasserstoff bedeuten kann,
W ein substituierter oder unsubstituierter Kohlenwasserstoffrest ist, der an Y gebunden ist und dessen Kette unterbrochen sein kann durch -S-, -O-, -NH-, -NR⁴-, -C(O)O-, -NHC(O)-, -C(O)NH-, -NHC(O)O-, -C(O)NHC(O)-, -NHC(O)NH-, -S(O)-, -C(S)O-, -C(S)NH-, -NHC(S)-, -NHC(S)O-,
Ar ein Rest ist, der mindestens eine aromatische Gruppe trägt, die unsubstituiert ist oder die mit einem oder mehreren Resten substituiert ist, wobei die aromatischen Gruppen frei sind von Substituenten, die ausgewählt sind unter der Boronsäure-, der Carbonsäure-, der Phosphinsäure-, der Phosphonsäure und der Sulfonsäuregruppe sowie von Hydroxygruppen,
wobei Ar an W gebunden sein muss, sofern a ≠ 0 ist,
der Index a gleich 0 oder 1 oder 2 ist,
der Index b = 1, 2, 3 oder eine ganze Zahl von mehr als 3 ist, wenn a ≠ 0 ist, und b = 1 oder 2 ist, wenn a = 1 ist,
der Index m 1, 2 oder eine ganze Zahl von mehr als 2 ist,
der Index n 0, 1, 2 oder größer 2 ist und
der Index o 1 oder 2 oder größer 2 ist.

2. Verbindung der Formel (2) oder Kieselsäure(hetero)polykondensat, umfassend Strukturen der Formel (2) 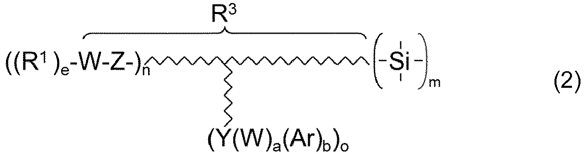 worin
die Reste und Substituenten R³, Y, W, Ar, a, b, m, n und o sowie die nicht näher gekennzeichneten Bindungen des Si-Atoms wie in Formel (1) des Anspruchs 1 definiert sind, Z ausgewählt ist unter Urethan-, Säureamid-, Ether und Estergruppen, R¹ eine geradkettige oder verzweigte, organisch polymerisierbaren Gruppe ist, die mindestens eine C=C-Doppelbindung trägt, und e 1, 2, 3, 4 oder eine ganze Zahl von mehr als 4 ist.

3. Verbindung der Formel (3) oder Kieselsäure(hetero)polykondensat, umfassend Strukturen der Formel (3), 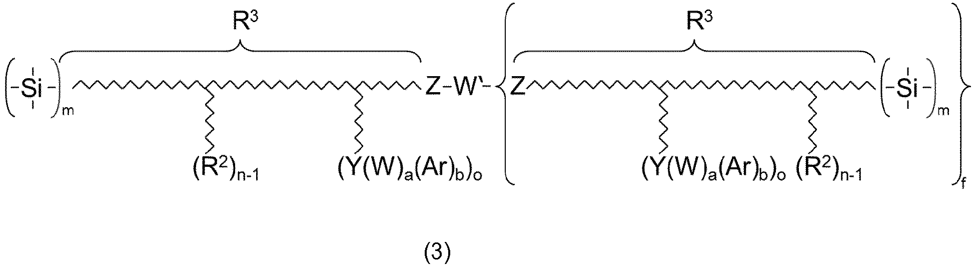 worin
die Reste und Substituenten R³, Y, W, Ar, Z, a, b, m, n und o sowie die nicht näher gekennzeichneten Bindungen des Si-Atoms wie in den Ansprüchen 1 und 2 definiert sind, W' für den Fall, dass beide Gruppen Z Estergruppen darstellen, die über das Carboxyl-Kohlenstoffatom an W' gebunden vorliegen, eine Einfachbindung sein kann und im Übrigen eine ggf. durch Sauerstoffatome, Schwefelatome, Carbonylgruppen, Carboxylgruppen, Aminogruppen oder Amidgruppen unterbrochene Kohlenstoffkette ist, und f = 1, 2, 3, 4, 5 oder 6 oder größer als 6 ist.

4. Verbindungsgemisch oder Kieselsäure(hetero)polykondensat, umfassend eine Verbindung oder Strukturen wie in Formel (1) des Anspruchs 1 angegeben, sowie eine Verbindung oder Strukturen der Formel (2) wie in Anspruch 2 definiert.

5. Verbindungsgemisch oder Kieselsäure(hetero)polykondensat nach Anspruch 4, umfassend weiterhin eine Verbindung oder Strukturen der Formel (3) wie in Anspruch 3 definiert.

6. Organisch vernetztes Kieselsäure(hetero)polykondensat, umfassend Strukturen der Formel (2) wie in Anspruch 2 definiert, in denen ein Teil oder alle Reste R¹ über eine Polymerisation miteinander vernetzt sind.

7. Organisch vernetztes Kieselsäure(hetero)polykondensat nach Anspruch 6, weiterhin umfassend Strukturen der Formel (0) 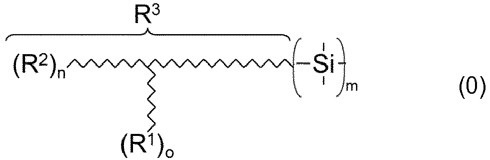 worin die Reste und Substituenten R², R³, o und n dieselbe Bedeutung wie in Formel (1) gemäß Anspruch 1 besitzen und R¹ dieselbe Bedeutung wie in Formel (2) des Anspruchs 2 besitzt, mit der Maßgabe, dass ein Teil oder alle Reste R¹ über eine Polymerisation oder über eine Michael-Addition mit Di- oder höheren Thiolen oder Di- oder höheren Aminen miteinander bzw. mit Resten R¹ der Struktur der Formel (2) miteinander vernetzt sind.

8. Komposit, umfassend eine Matrix aus einem gegebenenfalls organisch vernetzten Kieselsäure(hetero)polykondensat wie in einem der voranstehenden Ansprüche definiert sowie einen darin dispergierten, partikelförmigen Füllstoff.

9. Verfahren zum Herstellen eines Silans oder Kieselsäure(hetero)polykondensats nach Anspruch 1, umfassend die Schritte:
Bereitstellen eines Silans oder eines Kieselsäure(hetero)polykondensats mit der Struktur der Formel (0), 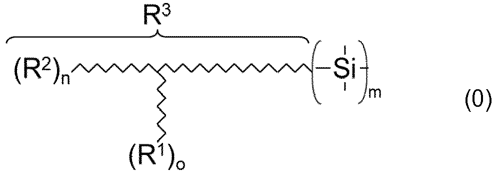 worin die Reste und Substituenten R², R³, m, n und o dieselbe Bedeutung wie in Formel (1) gemäß Anspruch 1 besitzen, R¹ zwei bis 50 Kohlenstoffatome aufweist und mindestens eine C=C-Doppelbindung besitzt. und
Umsetzen dieses Silans oder Kieselsäure(hetero)polykondensats mit einer Verbindung der Formel (II)
X-(W)ₐ(Ar)_{b} (II)
worin die Gruppen, Reste und Indices W, Ar, a und b die für Formel (1) in Anspruch 1 angegebenen Bedeutungen besitzen und X ausgewählt ist unter HS-, HNR⁴-, HP(O)R⁴_{c}Z'_{d}-, wobei Z' = OR⁴ und R⁴ die für Formel (1) angegebene Bedeutung besitzt, c = 0 oder 1, d = 0 oder 1 und (c+d) = 1 ist, HN= und HP(O)=.

10. Verfahren zum Herstellen eines Silans oder Kieselsäure(hetero)polykondensats nach Anspruch 2, umfassend die Schritte:
Bereitstellen eines Silans oder eines Kieselsäure(hetero)polykondensats mit der Struktur der Formel (1), 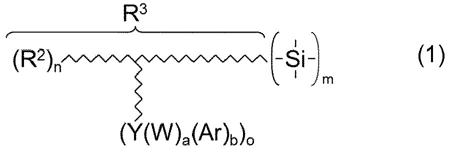 worin die Gruppen, Reste und Indices dieselbe Bedeutung haben wir für Struktur (1) in Anspruch 1 angegeben, und
Umsetzen dieses Silans oder Kieselsäure(hetero)polykondensats mit einer Verbindung der Formel (III)
Q-W(R¹)_{b} (III)
worin Q ausgewählt ist unter einer Isocyanatgruppe, einer Epoxygruppe sowie unter der Bedingung, dass R² in Formel (1) eine OH-Gruppe ist, einer Carbonsäuregruppe -COOH, die in aktivierter Form vorliegen kann, und unter der Bedingung, dass R² eine Carbonsäuregruppe oder ein Salz oder ein Ester davon ist, einer OH-Gruppe, W ein substituierter oder unsubstituierter Kohlenwasserstoffrest ist, dessen Kette unterbrochen sein kann durch -S-, -O-, -NH-, -NR⁴⁻, -C(O)O-, -NHC(O)-, -C(O)NH-, -NHC(O)O-, -C(O)NHC(O)-, -NHC(O)NH-, -S(O)-, -C(S)O-, -C(S)NH-, -NHC(S)-, -NHC(S)O-,
R¹ eine geradkettige oder verzweigte, organisch polymerisierbaren Gruppe ist, die mindestens eine C=C-Doppelbindung trägt, und
b 1, 2, 3, oder eine ganze Zahl größer 3 ist,
wobei die Verbindung der Formel (III) in einer stöchiometrischen oderunter- oder überstöchiometrischen Menge eingesetzt wird, bezogen auf den molaren Anteil an R².

11. Verfahren zum Herstellen eines Silans oder Kieselsäure(hetero)polykondensats nach Anspruch 3, umfassend die Schritte:
Bereitstellen eines Silans oder eines Kieselsäure(hetero)polykondensats mit der Struktur der Formel (1), 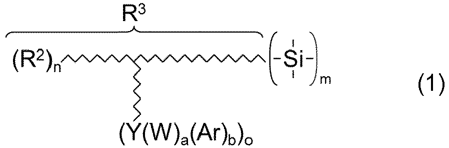 worin die Gruppen, Reste und Indices dieselbe Bedeutung haben wir für Struktur (1) in Anspruch 1 angegeben, und
Umsetzen dieses Silans oder Kieselsäure(hetero)polykondensats mit einer Verbindung der Formel (IV)
Q-W'[-Q]_{f} (IV)
worin Q ausgewählt ist unter einer Isocyanatgruppe, einer Epoxygruppe sowie, unter der Bedingung, dass R² in Formel (1) eine OH-Gruppe ist, einer Carbonsäuregruppe -COOH oder einer davon abgeleiteten aktivierten Carbonylverbindung, und unter der Bedingung,
dass R² eine Carbonsäuregruppe ist, einer OH-Gruppe, oder worin zwei Reste Q zusammen Teil eines cyclischen, intramolekularen Anhydrids einer Dicarbonsäure (-C(O)O(O)C-),
W' entweder ein substituierter oder unsubstituierter Kohlenwasserstoffrest ist, dessen Kette unterbrochen sein kann durch -S-, -O-, -NH-, -NR⁴-, -C(O)O-, -NHC(O)-, -C(O)NH-, -NHC(O)O-, -C(O)NHC(O)-, -NHC(O)NH-, -S(O)-, -C(S)O-, -C(S)NH-, -NHC(S)-, und/oder-NHC(S)O-, oder, unter der Bedingung, dass Q jeweils eine aktivierte Carbonsäuregruppe ist und c gleich 1 ist, eine Einfachbindung darstellt, wobei dann, wenn Q Teil eines cyclischen intramolekularen Anhydrids einer Dicarbonsäure ist, W' über zwei Kohlenstoffatome mit der Gruppe -C(O)O(O)C- verbunden ist, und f 1, 2, 3, 4, 5 oder 6 ist
wobei die Verbindung der Formel (III) in einem derartigen stöchiometrischen Verhältnis eingesetzt wird, dass die Anzahl der Reste R² und die Anzahl der Gruppen Q im Verhältnis von 1:1 oder ein Überschuss an Gruppen Q vorliegt.

12. Verfahren zum Herstellen eines Verbindungsgemisch oder Kieselsäure(hetero)polykondensats wie in Anspruch 4 angegeben, umfassend die Schritte:
Bereitstellen eines Silans oder eines Kieselsäure(hetero)polykondensats mit der Struktur der Formel (1), 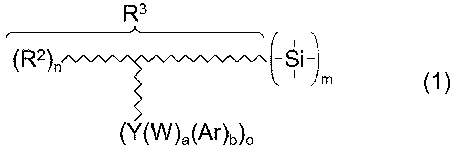 worin die Gruppen, Reste und Indices dieselbe Bedeutung haben wir für Struktur (1) in Anspruch 1 angegeben, und
Umsetzen dieses Silans oder Kieselsäure(hetero)polykondensats mit einer Verbindung der Formel (III)
Q-W(R¹)_{b} (III)
worin Q ausgewählt ist unter einer Isocyanatgruppe, einer Epoxygruppe sowie unter der Bedingung, dass R² in Formel (1) eine OH-Gruppe ist, einer Carbonsäuregruppe -COOH, die in aktivierter Form vorliegen kann, und unter der Bedingung, dass R² eine Carbonsäuregruppe oder ein Salz oder ein Ester davon ist, einer OH-Gruppe,
W ein substituierter oder unsubstituierter Kohlenwasserstoffrest ist, dessen Kette unterbrochen sein kann durch -S-, -O-, -NH-, -NR⁴-, -C(O)O-, -NHC(O)-, -C(O)NH-, -NHC(O)O-, -C(O)NHC(O)-, -NHC(O)NH-, -S(O)-, -C(S)O-, -C(S)NH-, -NHC(S)-, -NHC(S)O-,
R¹ eine geradkettige oder verzweigte, organisch polymerisierbaren Gruppe ist, die mindestens eine C=C-Doppelbindung trägt, und
b 1, 2, 3, oder eine ganze Zahl größer 3 ist,
wobei die Verbindung der Formel (III) in einer unterstöchiometrischen Menge eingesetzt wird, bezogen auf den molaren Anteil an R².

13. Verfahren zur Herstellung eines Verbindungsgemischs oder Kieselsäure(hetero)polykondensats nach Anspruch 5, umfassend die Schritte: Bereitstellen eines Silans oder eines Kieselsäure(hetero)polykondensats mit der Struktur der Formel (1), 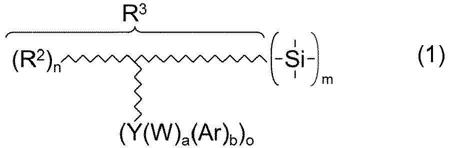 worin die Gruppen, Reste und Indices dieselbe Bedeutung haben wir für Struktur (1) in Anspruch 1 angegeben, und
Umsetzen dieses Silans oder Kieselsäure(hetero)polykondensats mit einer Verbindung der Formel (IV)
Q-W'[-Q]_{c} (IV)
worin Q ausgewählt ist unter einer Isocyanatgruppe, einer Epoxygruppe sowie, unter der Bedingung, dass R² in Formel (1) eine OH-Gruppe ist, einer Carbonsäuregruppe -COOH oder einer davon abgeleiteten aktivierten Carbonylverbindung, und unter der Bedingung, dass R² eine Carbonsäuregruppe ist, einer OH-Gruppe, oder worin zwei Reste Q zusammen Teil eines cyclischen, intramolekularen Anhydrids einer Dicarbonsäure (-C(O)O(O)C-),
W' entweder ein substituierter oder unsubstituierter Kohlenwasserstoffrest ist, dessen Kette unterbrochen sein kann durch -S-, -O-, -NH-, -NR⁴⁻, -C(O)O-, -NHC(O)-, -C(O)NH-, -NHC(O)O-, -C(O)NHC(O)-, -NHC(O)NH-, -S(O)-, -C(S)O-, -C(S)NH-, -NHC(S)-, und/oder-NHC(S)O-, oder, unter der Bedingung, dass Q jeweils eine aktivierte Carbonsäuregruppe ist und c gleich 1 ist, eine Einfachbindung darstellt, wobei dann, wenn Q Teil eines cyclischen intramolekularen Anhydrids einer Dicarbonsäure ist,
W' über zwei Kohlenstoffatome mit der Gruppe -C(O)O(O)C- verbunden ist, und f 1, 2, 3, 4, 5 oder 6 ist, wobei die Verbindung der Formel (IV) in einem derartigen stöchiometrischen Verhältnis eingesetzt wird, dass die Anzahl der Reste R² diejenige der Gruppen Q übersteigt.

14. Verfahren zum Herstellen eines Verbindungsgemischs oder Kieselsäure(hetero)polykondensats nach Anspruch 5, umfassend die Schritte: Bereitstellen eines Silans oder eines Kieselsäure(hetero)polykondensats mit der Struktur der Formel (1), 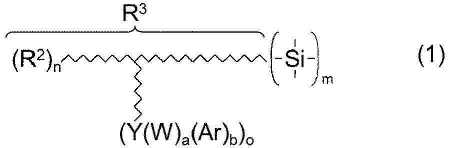 worin die Gruppen, Reste und Indices dieselbe Bedeutung haben wir für Struktur (1) in Anspruch 1 angegeben,
Umsetzen dieses Verbindungsgemischs oder Kieselsäure(hetero)polykondensats mit einer Verbindung der Formel (III)
Q-W(R¹)_{b} (III)
worin Q ausgewählt ist unter einer Isocyanatgruppe, einer Epoxygruppe sowie unter der Bedingung, dass R² in Formel (1) eine OH-Gruppe ist, einer Carbonsäuregruppe -COOH, die in aktivierter Form vorliegen kann, und unter der Bedingung, dass R² eine Carbonsäuregruppe oder ein Salz oder ein Ester davon ist, einer OH-Gruppe, W ein substituierter oder unsubstituierter Kohlenwasserstoffrest ist, dessen Kette unterbrochen sein kann durch -S-, -O-, -NH-, -NR⁴-, -C(O)O-, -NHC(O)-, -C(O)NH-, -NHC(O)O-, -C(O)NHC(O)-, -NHC(O)NH-, -S(O)-, -C(S)O-, -C(S)NH-, -NHC(S)-, -NHC(S)O-,
R¹ eine geradkettige oder verzweigte, organisch polymerisierbaren Gruppe ist, die mindestens eine C=C-Doppelbindung trägt, und
b 1, 2, 3, oder eine ganze Zahl größer 3 ist,
in einer molaren Menge α, und
Umsetzen dieses Verbindungsgemischs oder Kieselsäure(hetero)polykondensats mit einer Verbindung der Formel (IV)
Q-W'[-Q]_{f} (IV)
worin Q ausgewählt ist unter einer Isocyanatgruppe, einer Epoxygruppe sowie, unter der Bedingung, dass R² in Formel (1) eine OH-Gruppe ist, einer Carbonsäuregruppe -COOH oder einer davon abgeleiteten aktivierten Carbonylverbindung, und unter der Bedingung, dass R² eine Carbonsäuregruppe ist, einer OH-Gruppe, oder worin zwei Reste Q zusammen Teil eines cyclischen, intramolekularen Anhydrids einer Dicarbonsäure (-C(O)O(O)C-),
W' entweder ein substituierter oder unsubstituierter Kohlenwasserstoffrest ist, dessen Kette unterbrochen sein kann durch -S-, -O-, -NH-, -NR⁴-, -C(O)O-, -NHC(O)-, -C(O)NH-, -NHC(O)O-, -C(O)NHC(O)-, -NHC(O)NH-, -S(O)-, -C(S)O-, -C(S)NH-, -NHC(S)-, und/oder-NHC(S)O-, oder, unter der Bedingung, dass Q jeweils eine aktivierte Carbonsäuregruppe ist und c gleich 1 ist, eine Einfachbindung darstellt, wobei dann, wenn Q Teil eines cyclischen intramolekularen Anhydrids einer Dicarbonsäure ist,
W' über zwei Kohlenstoffatome mit der Gruppe -C(O)O(O)C- verbunden ist, und f 1, 2, 3, 4, 5 oder 6 ist,
in einer molaren Menge β/(f+1), bezogen auf den Index f in der Formel (IV), wobei die molaren Mengen so gewählt werden, dass die Bedingung α+β/(f+1) < n erfüllt ist, wobei der Index n die molare Menge an Gruppen R² in der eingesetzten Menge an Verbindung bzw. Kieselsäure(hetero)polykondensat der Struktur (1) angibt.

15. Verwendung eines Silans oder Kieselsäure(hetero)polykondensats oder eines Komposits wie in einem der Ansprüche 1 bis 8 definiert im Bereich der Optik oder für dentale Zwecke.

## Claims

1. A compound of the formula (1) or silicic acid (hetero)polycondensate comprising hydrolytically condensable structures of the formula (1), 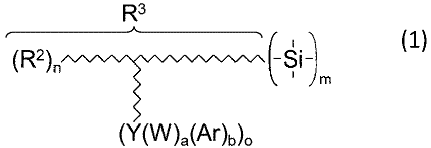 wherein
R² is a hydroxy group or a free carboxylic acid residue or a carboxylic acid ester or a salt derived therefrom,
R³ denotes a radical having a hydrocarbon-containing, branched or unbranched backbone which is bonded to the silicon via carbon and may be interrupted at will by heteroatoms or coupling groups or other groups containing heteroatoms, the zigzag line designating the hydrocarbon-containing backbone purely schematically, the three unspecified bonds of the Si atom representing further radicals bonded to the silicon atom, selected from radicals which can be hydrolysed from silicon, hydroxy groups and radicals bonded to the silicon via a carbon atom, which may have the same meaning as R³ or a different meaning, or which represent oxygen bridges to other silicon atoms and/or other metal atoms, if the structure (1) is part of a silicic acid (hetero)polycondensate,
the radical Y is either divalent and then has the meaning -S-, -NR⁴- or -P(O)(R⁴)_{c}(Z')_{d}-with Z' = OR⁴, c = 0 or 1, d = 0 or 1 and (c+d) = 1, or is trivalent and denotes -N= or - P(O)=,
R⁴ represents a hydrocarbon-containing radical and can also represent hydrogen in the radical -NR⁴⁻,
W is a substituted or unsubstituted hydrocarbon radical which is bonded to Y and whose chain may be interrupted by -S-, -0-, -NH-, -NR⁴-, -C(O)O-, -NHC(O)-, -C(O)NH-, -NHC(O)O-, -C(O)NHC(O)-, -NHC(O)NH-, -S(O)-, -C(S)O-, -C(S)NH-, -NHC(S)-, - NHC(S)O-,
Ar is a radical carrying at least one aromatic group which is unsubstituted or which is substituted by one or more radicals, the aromatic groups being free from substituents chosen from the boronic acid group, the carboxylic acid group, the phosphinic acid group, the phosphonic acid group and the sulphonic acid group and from hydroxy groups,
where Ar must be bound to W if a # 0,
the index a is 0 or 1 or 2,
the index b = 1, 2, 3 or an integer greater than 3 if a ≠ 0, and b = 1 or 2 if a = 1,
the index m is 1, 2 or an integer greater than 2,
the index n is 0, 1, 2 or greater than 2 and
the index o is 1 or 2 or greater than 2.

2. The compound of formula (2) or silicic acid (hetero)polycondensate comprising structures of formula (2) 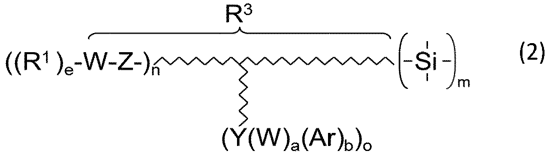 wherein the radicals and substituents R³, Y, W, Ar, a, b, m, n and o and the unspecified bonds of the Si atom are as defined in formula (1) of claim 1, Z is selected from urethane, acid amide, ether and ester groups, R¹ is a straight-chain or branched, organically polymerizable group which carries at least one C=C double bond, and e is 1, 2, 3, 4 or an integer greater than 4.

3. The compound of formula (3) or silicic acid (hetero)polycondensate comprising structures of formula (3), 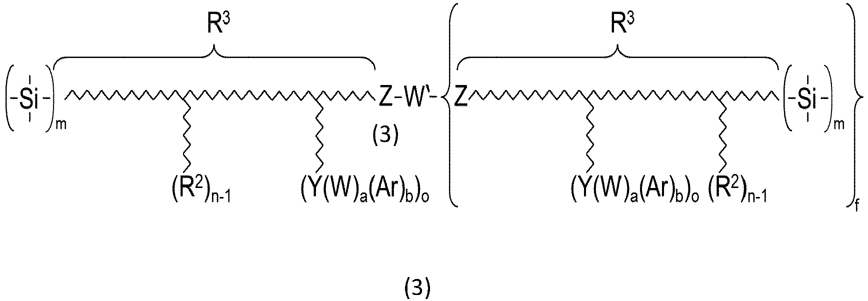 wherein the radicals and substituents R³, Y, W, Ar, Z, a, b, m, n and o and the unspecified bonds of the Si atom are as defined in Claims 1 and 2, W' can be a single bond in the case where both groups Z are ester groups bonded to W' via the carboxyl carbon atom, and is otherwise a carbon chain optionally interrupted by oxygen atoms, sulphur atoms, carbonyl groups, carboxyl groups, amino groups or amide groups, and f = 1, 2, 3, 4, 5 or 6 or greater than 6.

4. A compound mixture or silica (hetero)polycondensate comprising a compound or structures as defined in formula (1) of claim 1 and a compound or structures of formula (2) as defined in claim 2.

5. The compound mixture or silica (hetero)polycondensate according to claim 4, further comprising a compound or structures of formula (3) as defined in claim 3.

6. An organically crosslinked silica (hetero)polycondensate comprising structures of the formula (2) as defined in claim 2, in which some or all of the radicals R¹ are crosslinked with one another via a polymerization.

7. An organically crosslinked silica (hetero)polycondensate according to claim 6, further comprising structures of formula (0) 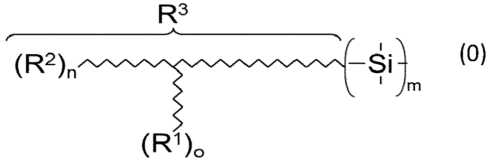 in which the radicals and substituents R², R³, o and n have the same meaning as in formula (1) according to claim 1 and R¹ has the same meaning as in formula (2) of claim 2, with the proviso that some or all of the radicals R¹ are crosslinked with one another or with radicals R¹ of the structure of formula (2) via a polymerization or via a Michael addition with di- or higher thiols or di- or higher amines.

8. A composite comprising a matrix of an optionally organically crosslinked silica (hetero)polycondensate as defined in any of the foregoing claims and a particulate filler dispersed therein.

9. A process for preparing a silane or silica (hetero)polycondensate according to claim 1, comprising the steps of
providing a silane or a silicic acid (hetero)polycondensate having the structure of formula (0), 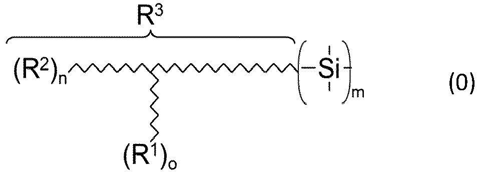 wherein the residues and substituents R², R³, m, n and o have the same meaning as in formula (1) according to claim 1, R¹ has two to 50 carbon atoms and has at least one C=C double bond, and
reacting this silane or silicic acid (hetero)polycondensate with a compound of formula (II)
X-(W)ₐ(Ar)_{b} (II)
in which the groups, radicals and indices W, Ar, a and b have the meanings given for formula (1) in claim 1 and X is chosen from HS-, HNR⁴-, HP(O)R⁴_{c}Z'_{d}-, where Z' = OR⁴ and R⁴ has the meaning given for formula (1) c, = 0 or 1, d = 0 or 1 and (c+d) = 1, HN= and HP(O) =.

10. A process for preparing a silane or silicic acid (hetero)polycondensate according to claim 2, comprising the steps of
providing a silane or a silicic acid (hetero)polycondensate having the structure of formula (1), 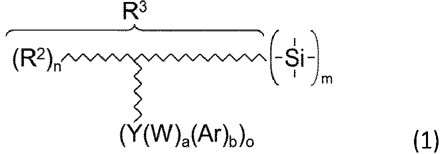 wherein the groups, residues and indices have the same meaning as given for structure (1) in claim 1, and
reacting this silane or silicic acid (hetero)polycondensate with a compound of formula (III)
Q-W(R¹)_{b} (III)
wherein Q is selected from an isocyanate group, an epoxy group and, provided that R² in formula (1) is an OH group, a carboxylic acid group -COOH which may be in activated form, and, provided that R² is a carboxylic acid group or a salt or ester thereof, an OH group, W is a substituted or unsubstituted hydrocarbon radical whose chain may be interrupted by -S-, -O-, -NH-, -NR⁴-, -C(O)O-, -NHC(O)-, -C(O)NH-, - NHC(O)O-, -C(O)NHC(O)-, -NHC(O)NH-, -S(O)-, -C(S)O-, C(S)NH-, -NHC(S)-, -NHC(S)O-, R¹ is a straight-chain or branched, organically polymerizable group which carries at least one C=C double bond, and b is 1, 2, 3, or an integer greater than 3, the compound of the formula (III) being used in a stoichiometric or below or above stoichiometric amount, based on the molar proportion of R².

11. A process for preparing a silane or silicic acid (hetero)polycondensate according to claim 3, comprising the steps of
providing a silane or a silicic acid (hetero)polycondensate having the structure of formula (1), 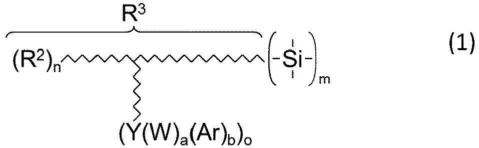 wherein the groups, residues and indices have the same meaning as given for structure (1) in claim 1, and
reacting this silane or silicic acid (hetero)polycondensate with a compound of formula (IV)
Q-W'[-Q]_{f} (IV)
wherein Q is selected from an isocyanate group, an epoxy group and, provided that R² in formula (1) is an OH group, a carboxylic acid group -COOH or an activated carbonyl compound derived therefrom, and, provided that R² is a carboxylic acid group, an OH group, or wherein two Q's together form part of a cyclic intramolecular anhydride of a dicarboxylic acid (-C(O)O(O)C-),
W' is either a substituted or unsubstituted hydrocarbon radical whose chain may be interrupted by -S-, -0-, -NH-, -NR⁴-, -C(O)O-, -NHC(O)-, -C(O)NH-, -NHC(O)O-, - C(O)NHC(O)-, -NHC(O)NH-, -S(O)-, -C(S)O-, -C(S)NH-, -NHC(S)-, and/or-NHC(S)O-, or, provided that each Q is an activated carboxylic acid group and c is 1, represents a single bond, wherein, when Q is part of a cyclic intramolecular anhydride of a dicarboxylic acid, W' is connected to the group -C(O)O(O)C- via two carbon atoms, and f is 1, 2, 3, 4, 5 or 6,
the compound of formula (III) being used in such a stoichiometric ratio that the number of radicals R² and the number of groups Q is in a ratio of 1:1 or an excess of groups Q.

12. A process for preparing a compound mixture or silica (hetero)polycondensate as claimed in claim 4, comprising the steps of
providing a silane or a silicic acid (hetero)polycondensate having the structure of formula (1), 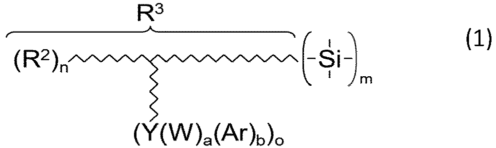 wherein the groups, residues and indices have the same meaning as given for structure (1) in claim 1, and
reacting this silane or silicic acid (hetero)polycondensate with a compound of formula (III)
Q-W(R¹)_{b} (III)
wherein Q is selected from an isocyanate group, an epoxy group and, provided that R² in formula (1) is an OH group, a carboxylic acid group -COOH which may be in activated form, and, provided that R² is a carboxylic acid group or a salt or ester thereof, an OH group, W is a substituted or unsubstituted hydrocarbon radical whose chain may be interrupted by -S-, -O-, -NH-, -NR⁴-, -C(O)O-, -NHC(O)-, -C(O)NH-, -NHC(O)O-, C(O)NHC(O)-, -NHC(O)NH, -S(O)-, -C(S)O-, -C(S)NH-, -NHC(S)-, -NHC(S)O-, R¹ is a straight-chain or branched, organically polymerizable group which carries at least one C=C double bond, and b is 1, 2, 3, or an integer greater than 3,
the compound of formula (III) being used in a sub-stoichiometric amount, based on the molar proportion of R².

13. A process for preparing a compound mixture or silica (hetero)polycondensate according to claim 5, comprising the steps of
providing a silane or a silicic acid (hetero)polycondensate having the structure of formula (1), 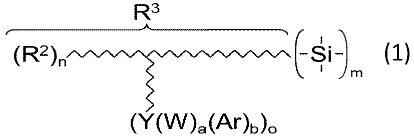 wherein the groups, residues and indices have the same meaning as given for structure (1) in claim 1, and
reacting this silane or silicic acid (hetero)polycondensate with a compound of formula (IV)
Q-W'[-Q]_{c} (IV)
wherein Q is selected from an isocyanate group, an epoxy group and, provided that R² in formula (1) is an OH group, a carboxylic acid group -COOH or an activated carbonyl compound derived therefrom, and, provided that R² is a carboxylic acid group, an OH group, or two Q's together form part of a cyclic intramolecular anhydride of a dicarboxylic acid (-C(O)O(O)C-),
W' is either a substituted or unsubstituted hydrocarbon radical whose chain may be interrupted by -S-, -0-, -NH-, -NR⁴-, -C(O)O-, -NHC(O)-, -C(O)NH-, -NHC(O)O-, - C(O)NHC(O)-, -NHC(O)NH-, -S(O)-, -C(S)O-, -C(S)NH-, -NHC(S)-, and/or -NHC(S)O-, or, provided that each Q is an activated carboxylic acid group and c is 1, represents a single bond, wherein, when Q is part of a cyclic intramolecular anhydride of a dicarboxylic acid, W' is linked to the group -C(O)O(O)C- via two carbon atoms, and f is 1, 2, 3, 4, 5 or 6, the compound of formula (IV) being used in such a stoichiometric ratio that the number of radicals R² exceeds that of the groups Q.

14. A process for preparing a compound mixture or silica (hetero)polycondensate according to claim 5, comprising the steps of
providing a silane or a silicic acid (hetero)polycondensate having the structure of formula (1), 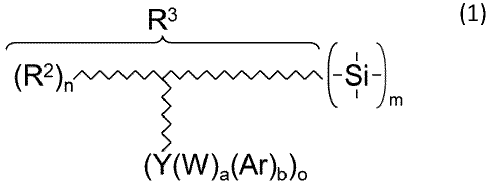 where the groups, radicals and indices have the same meaning as for structure (1) in claim 1 stated,
reacting this compound mixture or silicic acid (hetero)polycondensate with a compound of formula (III)
Q-W(R¹)_{b} (III)
wherein Q is selected from an isocyanate group, an epoxy group and, provided that R² in formula (1) is an OH group, a carboxylic acid group -COOH which may be in activeform, and, provided that R² is a carboxylic acid group or a salt or ester thereof, an OH group, W is a substituted or unsubstituted hydrocarbon radical whose chain may be interrupted by -S-, -O-, -NH-, -NR⁴-, -C(O)O-, -NHC(O)-, - C(O)NH-, -NHC(O)O-, -C(O)NHC(O)-, -NHC(O)NH-, -S(O)-, - C(S)O-, -C(S)NH-, -NHC(S)-, - NHC(S)O-,
R¹ is a straight-chain or branched, organically polymerizable group carrying at least one C=C double bond, and b is 1, 2, 3, or an integer greater than 3,
in a molar amount α, and
reacting this compound mixture or silicic acid (hetero)polycondensate with a compound of formula (IV)
Q-W'[-Q]_{f} (IV)
wherein Q is selected from an isocyanate group, an epoxy group and, provided that R² in formula (1) is an OH group, a carboxylic acid group -COOH or an activated carbonyl compound derived therefrom, and, provided that R² is a carboxylic acid group, an OH group, or two Q's together form part of a cyclic intramolecular anhydride of a dicarboxylic acid (-C(O)O(O)C-),
W' is either a substituted or unsubstituted hydrocarbon radical whose chain may be interrupted by -S-, -O-, -NH-, -NR⁴-, -C(O)O-, -NHC(O)-, -C(O)NH-, -NHC(O)O-, - C(O)NHC(O)-, -NHC(O)NH-, -S(O)-, -C(S)O-, C(S)NH-, -NHC(S)-, and/or-NHC(S)O-, or, provided that each Q is an activated carboxylic acid group and c is equal to 1, represents a single bond, wherein, when Q is part of a cyclic intramolecular anhydride of a dicarboxylic acid, W' is connected to the group -C(O)O(O)C- via two carbon atoms, and f is 1, 2, 3, 4, 5 or 6,
in a molar amount β/(f+1) with respect to the index f in formula (IV), said molar amounts being chosen so that the condition a+β(f+1) < n is satisfied, where the index n is the molar amount of groups R² in the amount of compound or silicic acid (hetero)polycondensate of structure (1).

15. Use of a silane or silicic acid (hetero)polycondensate or a composite as defined in one of claims 1 to 8 in the field of optics or for dental purposes.

## Revendications

1. Composé de la formule (1) ou (hétéro)polycondensat d'acide silicique, comprenant des structures de la formule (1) condensables par hydrolyse 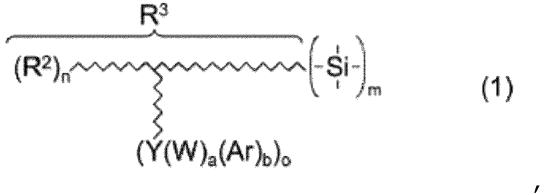 dans laquelle
R² est un groupe hydroxy ou un radical acide carboxylique libre ou un ester d'acide carboxylique dérivé de celui-ci ou un sel dérivé de celui-ci,
R³ désigne un radical avec une épine dorsale ramifiée ou non ramifiée, contenant un hydrocarbure, qui est liée au silicium par un carbone et qui peut être interrompue arbitrairement par des hétéroatomes ou des groupes de couplage ou d'autres groupes contenant des hétéroatomes, dans laquelle la ligne en zigzag désigne de façon purement schématique l'épine dorsale contenant un hydrocarbure, les trois liaisons non caractérisées plus en détail de l'atome de silicium représentent d'autres radicaux liés à l'atome de silicium, sélectionnés parmi des radicaux hydrolysables du silicium, des groupes hydroxy et des radicaux liés au silicium par un atome de carbone, qui peuvent avoir la même signification que R³ ou une signification différant de celle-ci ou représentent des ponts d'oxygène vers d'autres atomes de silicium et/ou d'autres atomes métalliques, lorsque la structure (1) est un composant d'un (hétéro)polycondensat d'acide silicique,
le radical Y soit est à liaison double et a alors la signification -S-, -NR⁴, ou -P(O) (R⁴)_{c}(Z')_{d}- avec Z' = OR⁴, c = 0 ou 1, d = 0 ou 1 et (c+d) = 1 soit est à liaison triple et signifie -N= ou -P(O)=,
R⁴ représente un radical contenant un hydrocarbure et peut dans le radical -NR⁴ signifier aussi en outre un hydrogène,
W est un radical substitué ou non substitué contenant un hydrocarbure, qui est lié à Y et dont la chaîne peut être interrompue par -S-, -O-, -NH-, -NR⁴-, -C(O)O-, -NHC(O)-, -C(O)NH-, -NHC(CO)O-, -C(O)NHC(O)-, -NHC(O)NH-, -S(O)-, -C(S)O-, -C(S)NH-, -NHC(S)-, -NHC(S)O-,
Ar est un radical, qui porte au moins un groupe aromatique, qui est non substitué ou qui est substitué avec un ou plusieurs radicaux, dans lequel les groupes aromatiques sont dépourvus de substituants, qui sont sélectionnés parmi les groupes acide borique, acide carboxylique, acide phosphinique, acide phosphonique et acide sulfonique, ainsi que de groupes hydroxy,
dans laquelle Ar doit être lié à W, dans la mesure où a ≠ 0,
l'indice a est égal à 0 ou 1 ou 2,
l'indice b = 1, 2 ou 3 ou un nombre entier supérieur à 3, lorsque a ≠ 0, et b = 1 ou 2 lorsque a = 1,
l'indice m est 1, 2 ou un nombre entier supérieur à 2,
l'indice n est 0, 1, 2 ou supérieur à 2, et
l'indice o est 1 ou 2 ou supérieur à 2.

2. Composé de la formule (2) ou (hétéro)polycondensat d'acide silicique comprenant des structures de la formule (2) 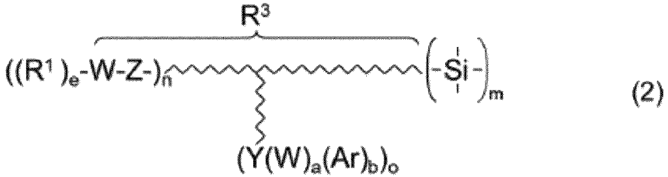 dans laquelle
les radicaux et substituants R³, Y, W, Ar, a, b, m, n et o ainsi que les liaisons de l'atome Si non caractérisées plus en détail sont définis de la même manière que dans la formule (1) de la revendication 1, Z est sélectionné parmi des groupes uréthane, amide d'acide, éther et ester, R¹ est un groupe à chaîne droite ou ramifié, organiquement polymérisable, qui porte au moins une liaison double C=C, et e est 1, 2, 3, 4 ou un nombre entier supérieur à 4.

3. Composé de la formule (3) ou (hétéro)polycondensat d'acide silicique, comprenant des structures de la formule (3), 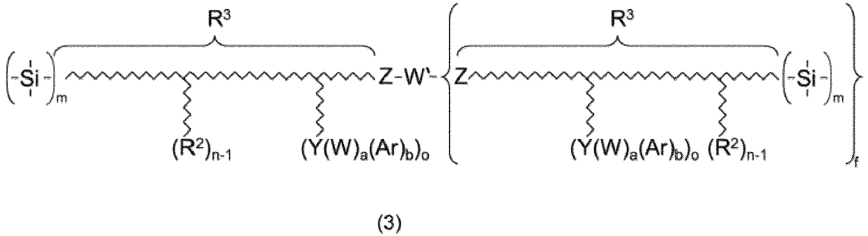 dans laquelle
les radicaux et substituants R³, Y, W, Ar, Z, a, b, m, n et o ainsi que les liaisons de l'atome Si non caractérisées plus en détail sont définis de la même manière que dans les revendications 1 et 2, W' peut, pour le cas où les deux groupes Z représentent des groupes ester, qui sont liés à W' par l'atome de carbone carboxyle, être une liaison simple et pour le reste est une chaîne de carbone éventuellement interrompue par des atomes d'oxygène, des atomes de soufre, des groupes carbonyle, des groupes carboxyle, des groupes aminés ou des groupes amide, et f = 1, 2, 3, 4, 5, 6 ou est supérieur à 6.

4. Mélange de composé ou (hétéro)polycondensat d'acide silicique, comprenant un composé ou des structures du type indiqué dans la formule (1) de la revendication 1, ainsi qu'un composé ou des structures de la formule (2) définie comme dans la revendication 2.

5. Mélange de composé ou (hétéro)polycondensat d'acide silicique selon la revendication 4, comprenant en outre un composé ou des structures de la formule (3) définie comme dans la revendication 3.

6. (Hétéro)polycondensat d'acide silicique organiquement réticulé, comprenant des structures de la formule (2) définie comme dans la revendication 2, dans lesquelles une partie ou tous les radicaux R¹ sont réticulés les uns avec les autres par une polymérisation.

7. (Hétéro)polycondensat d'acide silicique organiquement réticulé selon la revendication 6, comprenant en outre des structures de la formule (0) 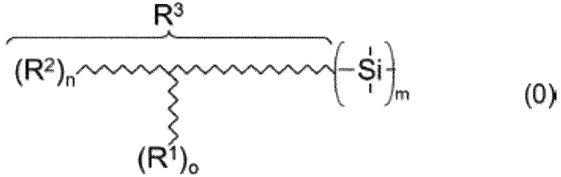 dans laquelle les radicaux et substituants R², R³, o et n ont la même signification que dans la formule (1) selon la revendication 1 et R¹ a la même signification que dans la formule (2) de la revendication 2, à la condition qu'une partie ou que tous les radicaux R¹ soient réticulés les uns avec les autres ou respectivement avec des radicaux R¹ de la structure de la formule (2) les uns avec les autres par une polymérisation ou par une addition de Michael avec des dithiols ou des thiols supérieurs ou des diamines ou des amines supérieures.

8. Composite, comprenant une matrice en un (hétéro)polycondensat d'acide silicique éventuellement organiquement réticulé du type défini dans l'une quelconque des revendications précédentes, ainsi qu'une matière de charge sous forme particulaire dispersée dans celle-ci.

9. Procédé de fabrication d'un silane ou d'un (hétéro)polycondensat d'acide silicique selon la revendication 1, comprenant les étapes suivantes:
préparer un silane ou un (hétéro)polycondensat d'acide silicique avec la structure de la formule(0) 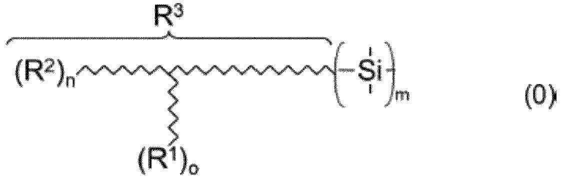 dans laquelle les radicaux et substituants R², R³, m, n et o ont la même signification que dans la formule (1) selon la revendication 1, R¹ présente deux à 50 atomes de carbone et possède au moins une liaison double C=C, et faire réagir ce silane ou cet (hétéro)polycondensat d'acide silicique avec un composé de la formule (II),
X-(W)ₐ(Ar)_{b} (II)
dans laquelle les groupes, radicaux et indices W, Ar, a et b ont les significations indiquées pour la formule (1) dans la revendication 1 et X est sélectionné parmi HS-, HNR⁴-, HP(O)R⁴_{c}Z'_{d}-, dans laquelle Z' = OR⁴ et R⁴ a la signification indiquée pour la formule (1), c = 0 ou 1, d = 0 ou 1 et (c+d) = 1, HN= et HP(0)=.

10. Procédé de fabrication d'un silane ou d'un (hétéro)polycondensat d'acide silicique selon la revendication 2, comprenant les étapes suivantes:
préparer un silane ou un (hétéro)polycondensat d'acide silicique avec la structure de la formule (1), 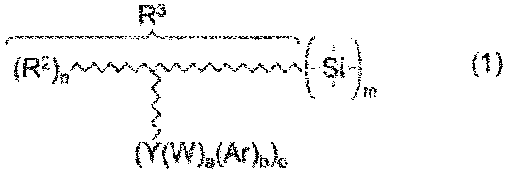 dans laquelle les groupes, radicaux et indices ont la même signification que pour la structure (1) indiquée dans la revendication 1, et
faire réagir ce silane ou cet (hétéro)polycondensat d'acide silicique avec un composé de la formule (III)
Q-W(R¹)_{b} (III)
dans laquelle Q est sélectionné parmi un groupe isocyanate, un groupe époxy ainsi que, sous la condition que R² dans la formule (1) soit un groupe OH, un groupe acide carboxylique-COOH, qui peut être présent sous forme activée, et sous la condition que R² soit un groupe acide carboxylique ou un sel ou un ester de celui-ci, un groupe OH, W est un radical hydrocarboné substitué ou non substitué, dont la chaîne peut être interrompue par -S-, -O-, -NH-, -NR⁴-, -C(O)O-, -NHC(O)-, -C(O)NH-, -NHC(O)O-, -C(O)NHC(O)-, -NHC(O)NH-, -S(O)-, -C(S)O-, -C(S)NH-,-NHC(S)-, -NHC(S)O-,
R¹ est un groupe à chaîne droite ou ramifié, organiquement polymérisable, qui porte au moins une liaison double C=C, et
b est 1, 2, 3 ou un nombre entier supérieur à 3, dans lequel on utilise le composé de la formule (III) en une quantité stœchiométrique ou sous- ou sur-stœchiométrique, rapportée à la proportion molaire de R².

11. Procédé de fabrication d'un silane ou d'un (hétéro)polycondensat d'acide silicique selon la revendication 3, comprenant les étapes suivantes:
préparer un silane ou un (hétéro)polycondensat d'acide silicique avec la structure de la formule (1), 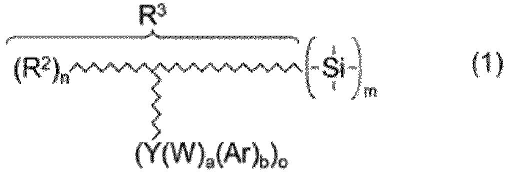 dans laquelle les groupes, radicaux et indices ont la même signification que pour la structure (1) indiquée dans la revendication 1, et
faire réagir ce silane ou cet (hétéro)polycondensat d'acide silicique avec un composé de la formule (IV)
**Q-W'[-Q]_{f}** **(IV)**
dans laquelle Q est sélectionné parmi un groupe isocyanate, un groupe époxy ainsi que, sous la condition que R² dans la formule (1) soit un groupe OH, un groupe acide carboxylique-COOH ou un composé carbonyle activé dérivé de celui-ci, et sous la condition que R² soit un groupe acide carboxylique, un groupe OH, ou dans laquelle deux radicaux Q ensemble font partie d'un anhydride intramoléculaire cyclique d'un acide dicarboxylique (-C(O)O(O)C-) ,
W' soit est un radical hydrocarboné substitué ou non substitué, dont la chaîne peut être interrompue par -S-, -O-, -NH-, -NR⁴-, -C(O)-, -NHC(O)-, -C(O)NH-, -NHC(O)O-, -C(O)NHC(O)-, -NHC(O)NH-, -S(O)-, -C(S)O-, -C(S)NH-,-NHC(S)- et/ou -NHC(S)O-, soit, sous la condition que Q soit chaque fois un groupe acide carboxylique activé et que c soit égal à 1, représente une liaison simple, dans lequel, lorsque Q est une partie d'un anhydride intramoléculaire cyclique d'un acide dicarboxylique, W' est lié par deux atomes de carbone au groupe -C(O)O(O)C-, et f est 1, 2, 3, 4, 5 ou 6,
dans lequel on utilise le composé de la formule (III) dans un rapport stœchiométrique tel que le nombre des radicaux R² et le nombre des groupes Q soient présents dans un rapport de 1:1 ou avec un excédent de groupes Q.

12. Procédé de fabrication d'un mélange de composé ou d'un (hétéro)polycondensat d'acide silicique tel qu'indiqué dans la revendication 4, comprenant les étapes suivantes: préparer un silane ou un (hétéro)polycondensat d'acide silicique avec la structure de la formule (1), 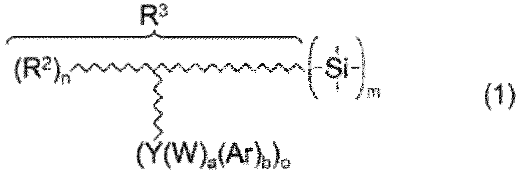 dans laquelle les groupes, radicaux et indices ont la même signification que pour la structure (1) indiquée dans la revendication 1, et
faire réagir ce silane ou cet (hétéro)polycondensat d'acide silicique avec un composé de la formule (III)
Q-W(R¹)_{b} (III)
dans laquelle Q est sélectionné parmi un groupe isocyanate, un groupe époxy ainsi que, sous la condition que R² dans la formule (1) soit un groupe OH, un groupe acide carboxylique-COOH, qui peut être présent sous forme activée, et sous la condition que R² soit un groupe acide carboxylique ou un sel ou un ester de celui-ci, un groupe OH,
W est un radical hydrocarboné substitué ou non substitué, dont la chaîne peut être interrompue par -S-, -O-, -NH-, -NR⁴-, -C(O)O-, -NHC(O)-, -C(O)NH-, -NHC(O)O-, - C(O)NHC(O)-, -NHC(O)NH-, -S(O)-, -C(S)O-, -C(S)NH-,-NHC(S)-, -NHC(S)O-,
R¹ est un groupe à chaîne droite ou ramifié, organiquement polymérisable, qui porte au moins une liaison double C=C, et
b est 1, 2, 3 ou un nombre entier supérieur à 3,
dans lequel on utilise le composé de la formule (III) en une quantité sous-stœchiométrique, rapportée à la proportion molaire de R².

13. Procédé de fabrication d'un mélange de composé ou d'un (hétéro)polycondensat d'acide silicique selon la revendication 5, comprenant les étapes suivantes:
préparer un silane ou un (hétéro)polycondensat d'acide silicique avec la structure de la formule (1), 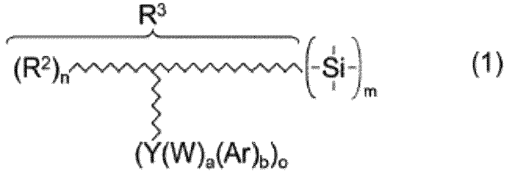 dans laquelle les groupes, radicaux et indices ont la même signification que pour la structure (1) indiquée dans la revendication 1, et
faire réagir ce silane ou cet (hétéro)polycondensat d'acide silicique avec un composé de la formule (IV)
**Q-W'[-Q]_{f}** **(IV)**
dans laquelle Q est sélectionné parmi un groupe isocyanate, un groupe époxy ainsi que, sous la condition que R² dans la formule (1) soit un groupe OH, un groupe acide carboxylique-COOH ou un composé carbonyle activé dérivé de celui-ci, et sous la condition que R² soit un groupe acide carboxylique, un groupe OH, ou dans laquelle deux radicaux Q ensemble font partie d'un anhydride intramoléculaire cyclique d'un acide dicarboxylique (-C(O)O(O)C-),
W' soit est un radical hydrocarboné substitué ou non substitué, dont la chaîne peut être interrompue par -S-, -O-, -NH-, -NR⁴-, -(O)O-, -NHC(O)-, -C(O)NH-, -NHC(O)O-, -C(O)NHC(O)-, -NHC(O)NH-, -S(O)-, -C(S)O-, -C(S)NH-,-NHC(S)- et/ou -NHC(S)O-, soit, sous la condition que Q soit chaque fois un groupe acide carboxylique activé et que c soit égal à 1, représente une liaison simple, dans lequel, lorsque Q est une partie d'un anhydride intramoléculaire cyclique d'un acide dicarboxylique, W' est lié par deux atomes de carbone au groupe -C(O)O(O)C-, et f est 1, 2, 3, 4, 5 ou 6,
dans lequel on utilise le composé de la formule (IV) dans un rapport stœchiométrique tel que le nombre des radicaux R² dépasse le nombre des groupes Q.

14. Procédé de fabrication d'un mélange de composé ou d'un (hétéro)polycondensat d'acide silicique selon la revendication 5, comprenant les étapes suivantes:
préparer un silane ou un (hétéro)polycondensat d'acide silicique la structure de la formule (1), 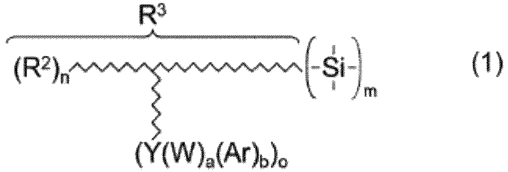 dans laquelle les groupes, radicaux et indices ont la même signification que pour la structure (1) indiquée dans la revendication 1,
faire réagir ce mélange de composé ou cet (hétéro)polycondensat d'acide silicique avec un composé de la formule (III)
**Q-W(R¹)_{b}** **(III)**
dans laquelle Q est sélectionné parmi un groupe isocyanate, un groupe époxy ainsi que, sous la condition que R² dans la formule (1) soit un groupe OH, un groupe acide carboxylique-COOH, qui peut être présent sous forme activée, et sous la condition que R² soit un groupe acide carboxylique ou un sel ou un ester de celui-ci, un groupe OH,
W est un radical hydrocarboné substitué ou non substitué, dont la chaîne peut être interrompue par -S-, -O-, -NH-, -NR⁴⁻, -C(O)O-, -NHC(O)-, -C(O)NH-, -NHC(O)O-,-C (O)NHC(O)-, -NHC(O)NH-, -S(O)-, -C(S)O- , -C(S)NH-,-NHC(S)-, -NHC(S)O-,
R¹ est un groupe à chaîne droite ou ramifié, organiquement polymérisable, qui porte au moins une liaison double C=C, et
b est 1, 2, 3 ou un nombre entier supérieur à 3, en une quantité molaire a, et
faire réagir ce mélange de composé ou cet (hétéro)polycondensat d'acide silicique avec un composé de la formule (IV)
**Q-W'[-Q]_{f}** **(IV)**
dans laquelle Q est sélectionné parmi un groupe isocyanate, un groupe époxy ainsi que, sous la condition que R² dans la formule (1) soit un groupe OH, un groupe acide carboxylique-COOH ou un composé carbonyle activé dérivé de celui-ci, et sous la condition que R² soit un groupe acide carboxylique, un groupe OH, ou dans laquelle deux radicaux Q ensemble font partie d'un anhydride intramoléculaire cyclique d'un acide dicarboxylique (-C(O)O(O)C-) ,
W' soit est un radical hydrocarboné substitué ou non substitué, dont la chaîne peut être interrompue par -S-, -O-, -NH-, -NR⁴-, -C(O)O-, -NHC(O)-, -C(O)NH-, -NHC(O)O-, -C(O)NHC(O)-, -NHC(O)NH-, -S(O)-, -C(S)O-, -C(S)NH-,-NHC(S)-, et/ou -NHC(S)O-, soit, sous la condition que Q soit chaque fois un groupe acide carboxylique activé et que c soit égal à 1, représente une liaison simple, dans lequel, lorsque Q est une partie d'un anhydride intramoléculaire cyclique d'un acide dicarboxylique, W' est lié par deux atomes de carbone au groupe -C(O)O(O)C-, et f est 1, 2, 3, 4, 5 ou 6,
en une quantité molaire β/(f+1), rapportée à l'indice f dans la formule (IV), dans lequel on choisit les quantités molaires, de telle manière que la condition α+β/f+1) < n soit remplie, l'indice n indiquant la quantité molaire de groupes R² dans la quantité utilisée de composé ou d'(hétéro)polycondensat d'acide silicique de la structure (1) .

15. Utilisation d'un silane ou d'un (hétéro)polycondensat d'acide silicique ou d'un composite défini comme dans l'une quelconque des revendications 1 à 8 dans le domaine de l'optique ou pour des applications dentaires.
